# FASCICULE DE BREVET EUROPEEN

(11) **EP 0 864 644 B1**
(45) Date de publication et mention de la délivrance du brevet: **19.10.2005**
(21) Numéro de dépôt: 98400093.5
(22) Date de dépôt: 20.01.1998
(51) Int. Cl.: C12N 1/20, A61K 39/108

(54) **Souches mutantes non pathogènes d'E.coli, leur procédé d'obtention et leurs utilisations**
Nicht pathogener mutanter E. coli Stamm, Verfahren zur dessen Herstellung und Verwendung
Non-pathogenic E.Coli mutant strain, process or their preparation and uses

(30) Priorité: 20.01.1997 FR 9700532
(43) Date de publication de la demande: 16.09.1998
(73) Titulaire: INSTITUT NATIONAL DE LA RECHERCHE AGRONOMIQUE (INRA), 75341 Paris Cédéx 07 (FR); Ecole Nationale Vétérinaire de Toulouse (ENVT), 31076 Toulouse Cedex (FR)
(72) Inventeur: Milon, Alain, 31700 Blagnac (FR); De Rycke, Jean, 31820 Pibrac (FR)
(74) Mandataire: Vialle-Presles, Marie José

(56) Documents cités:
- WIELER L H ET AL: "Shiga toxin-producing Escherichia coli strains from bovines: Association of adhesion with carriage of eae and other genes." JOURNAL OF CLINICAL MICROBIOLOGY 34 (12). 1996. 2980-2984, XP002042689
- MILON A ET AL: "ORAL VACCINATION OF WEANED RABBITS AGAINST ENTEROPATHOGENIC ESCHERICHIA- COLI LIKE ESCHERICHIA- COLI O103 INFECTION USE OF HETEROLOGOUS STRAINS HARBORING LIPOPOLYSACCHARIDE OR ADHESIN OF PATHOGENIC STRAINS." INFECT IMMUN 60 (7). 1992. 2702-2709, XP002042690
- PILLIEN F. ET AL.: "Role of Adhesive Factor/Rabbit 2 in experimental enteropathogenic Escherichia coli O103 diarrhea of weaned rabbit" VETERINARY MICROBIOLOGY, vol. 60, 1996, pages 105-115, XP002042691
- LEROY S. ET AL.: "Presence of eaeA sequences in pathogenic and non-pathogenic Escherichia coli strains isolated from weaned rabbits" JOURNAL OF MEDICAL MICROBIOLOGY, vol. 40, no. 2, février 1994, pages 90-94, XP002042692
- DATABASE BIOSIS BIOSCIENCES INFORMATION SERVICE, PHILADELPHIA, PA, US AGIN T S ET AL: "Characterization of the eaeA gene from rabbit enteropathogenic Escherichia coli strain RDEC-1 and comparison to other eaeA genes from bacteria that cause attaching- effacing lesions." XP002066596 & FEMS MICROBIOLOGY LETTERS 144 (2-3). 1996. 249-258,
- FIEDERLING F ET AL: "Adhesive factor-rabbit 2, a new fimbrial adhesin and a virulence factor from Escherichia coli O103, a serogroup enteropathogenic for rabbits." INFECTION AND IMMUNITY 65 (2). 1997. 847-851, XP002042693
- DE RYCKE J ET AL: "Enteropathogenic Escherichia coli O103 from rabbit elicits actin stress fibers and focal adhesions in HeLa epithelial cells, cytopathic effects that are linked to an analog of the locus of enterocyte effacement." INFECTION AND IMMUNITY, (1997 JUL) 65 (7) 2555-63, XP002066505

### LISTE DE SEQUENCES

(1) INFORMATIONS GENERALES:
   (i) DEPOSANT:
      (A) NOM: INSTITUT NATIONAL DE LA RECHERCHE AGRONOMIQUE
      (B) RUE: 147 RUE DE L'UNIVERSITE
      (C) VILLE: PARIS CEDEX 07
      (E) PAYS: FRANCE
      (F) CODE POSTAL: 75341

      (A) NOM: ECOLE NATIONALE VETERINAIRE DE TOULOUSE
      (B) RUE: 23, CHEMIN DES CAPELLES
      (C) VILLE: TOULOUSE CEDEX
      (E) PAYS: FRANCE
      (F) CODE POSTAL: 31076
   (ii) TITRE DE L' INVENTION: SOUCHES MUTANTES NON PATHOGENES D'E. COLI, LEUR PROCEDE D'OBTENTION ET LEURS UTILISATIONS
   (iii) NOMBRE DE SEQUENCES: 5
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 12626 paires de bases
      (B) TYPE: nucléotide
      (C) NOMBRE DE BRINS: double
      (D) CONFIGURATION: linéaire
   (ii) TYPE DE MOLECULE: ADN (génomique)
   (ix) CARACTERISTIQUE:
      (A) NOM/CLE: CDS
      (B) EMPLACEMENT:1768..4587
      (D) AUTRES INFORMATIONS:/product= "eaeA"
   (ix) CARACTERISTIQUE:
      (A) NOM/CLE: CDS
      (B) EMPLACEMENT:7278..7856
      (D) AUTRES INFORMATIONS:/product= "espA"
   (ix) CARACTERISTIQUE:
      (A) NOM/CLE: CDS
      (B) EMPLACEMENT:7869..9011
      (D) AUTRES INFORMATIONS:/product= "espD"
   (ix) CARACTERISTIQUE:
      (A) NOM/CLE: CDS
      (B) EMPLACEMENT:9032..9976
      (D) AUTRES INFORMATIONS:/product= "espB"
   (xi) DESCRIPTION DE LA SEQUENCE: SEQ ID NO: 1:
   GATATCGCCG AAGCCCGTCA GAGGATAATA GATAAATTAG AACCAAAGGA TACAAAGGAG      60
   ACGAAGGAGC CAGGGGATCC AAATAGTGGC GAGGGAAAAA TCATTGAAAT TCATACCTCA     120
   ACCTCAACTT CTAGCCTCCG TGCAGATCCT AAACTTTGGT TGTCATTGGG GACTATTGCT     180
   GCAGGTCTGA TAGGGATGGC TGCGACGGGG ATTGCACAGG CTGTTGCGTT GACTCCAGAG     240
   CCGGATGACC CAATCACTAC CGACCCTGAT GCTGCAGCAA ACACAGCTGA AGCAGCGGCA     300
   AAAGATCAGT TAACGAAAGA AGCATTCCAG AACCCAGATA ACCAGAAAGT TAATATCGAT     360
   GAGAACGGAA ATGCAATTCC GTCCGGGGAA CTAAAAGATG ATGTTGTTGC GCAAATAGCA     420
   GAACAAGCTA AAGCGGCGGG TGAACAGGCC AGACAGGAAG CTATTGAAAG TAATTCTCAG     480
   GCGCAGCAAA AATATGATGA ACAGCATGCT AAACGCGAAC AGGAAATGTC TCTTTCATCG     540
   GGGGTTGGCT ACGGTATTAG TGGTGCGCTG ATTCTTGGCG GGGGAATTGG TGCCGGTGTT     600
   ACTGCTGCTC TTCATCGGAA AAACCAACCG GCAGAACAAA CAATCACTAC ACGTACGGTA     660
   GTCGATAATC AGCCTACGAA TAACGCATCT GCGCAGGGCA ATACTGACAC AAGTGGGCCA     720
   GAAGAGTCCC CGGCGAGCAG ACGTAATTCG AATGCCAGCC TCGCATCGAA CGGGTCTGAC     780
   ACCTCCAGCA CGGGCACGGT AGAGAATCCG TATGCTGACG TTGGAATGCC CAGAAATGAT     840
   TCACTGGCTC GCATTTCAGA GGAACCTATT TATGATGAGG TCGCTGCAGA TCCTAATTAT     900
   AGCGTCATTC AACATTTTTC AGGGAACAGC CCAGTTACCG GAAGGTTAGT GGGAACCCCA     960
   GGGCAAGGTA TCCAAAGTAC TTATGCGCTT CTGGCAAGCA GCGGCGGATT GCGTTTAGGT    1020
   ATGGGAGGAT TAACGGGGGG GGGCGAGAGC GCAGTAAGTA CTGCCAATGC CGCACCAACG    1080
   CCGGGACCCG CACGTTTCGT TTAAATATAT CCGTGAGTAT TTAGTTGAGG TTGGGAGGGG    1140
   GGGAGTTTTA CTAATATTAA TGTTTCAGAG AATAAAGTTG CAGCATGGGT AACTCTTGAA    1200
   CTTCTGTAAT TATAAATTCA ATTAAGAGAA ATTATAATGT CATCAAGATC TGAGCTTTTA    1260
   TTAGATAGAT TTGCAGAAAA AATTGGTATT GGATCTATTT CATTTAATGA AAATAGATTG    1320
   TGTTCTTTTG CTATTGATGA GATTTATTAT ATTTCGTTAT CTGATGCTAA TGACGAATAT    1380
   ATGATGATCT ATGGCGTCTG TGGGAAATTC CCGACAGATA ATCCTAACTT CGCTCTTGAG    1440
   ATTTTGAATG CAAACTTATG GTTTGCAGAG AATGGTGGAC CATACCTGTG TTATGAATCA    1500
   GGAGCACAAT CGCTGTTGTT AGCGTTACGT TTTCCGCTCG ATGATGCTAC ACCTGAAAAA    1560
   CTTGAGAATG AAATAGAAGT CGTCGTTAAG TCAATGGAAA ACCTGTATTT GGTATTACAT    1620
   AATCAGGGAA TAACATTAGA AAACGAACAT ATGAAAATAG AGGAAATTAG TTCAAGCGAT     1680
   AATAAACATT ATTACGCCGG AAGATAAAAG ATTATTTATT AATATAATTT ACTATTCATT     1740
   CTAACTCATT GTGGTGGAGC CCATAAC ATG ATT ACT CAT GGT TTT TAT GCC         1791
                                 Met Ile Thr His Gly Phe Tyr Ala
                                   1               5
   CGG ACC CGG CAC AAG CAT AAG CTA AAA AAA ACA TTT ATT ATG CTT AGT      1839
   Arg Thr Arg His Lys His Lys Leu Lys Lys Thr Phe Ile Met Leu Ser
        10                  15                  20
   GCT GGT TTA GGA TTG TTT TTT TAT GTT AAC CAG AAT TCA TTT GCA AAT      1887
   Ala Gly Leu Gly Leu Phe Phe Tyr Val Asn Gln Asn Ser Phe Ala Asn
    25                  30                  35                  40
   GGT GAA AAT TAT TTT AAA TTG AGT TCA GAT TCA AAA CTG TTA ACT CAA      1935
   Gly Glu Asn Tyr Phe Lys Leu Ser Ser Asp Ser Lys Leu Leu Thr Gln
                    45                  50                  55
   AAT GCC GCT CAG GAT CGC CTT TTT TAT ACG TTA AAA ACA GGT GAA ACT      1983
   Asn Ala Ala Gln Asp Arg Leu Phe Tyr Thr Leu Lys Thr Gly Glu Thr
                60                  65                  70
   GTT GCC AAT ATT TCT AAA TCA CAG GGT ATC AGT TTA TCG GTA ATT TGG      2031
   Val Ala Asn Ile Ser Lys Ser Gln Gly Ile Ser Leu Ser Val Ile Trp
            75                  80                  85
   TCA CTG AAT AAA CAT TTA TAC AGT TCC GAA AGC GAA ATG ATG AAG GCT      2079
   Ser Leu Asn Lys His Leu Tyr Ser Ser Glu Ser Glu Met Met Lys Ala
        90                  95                 100
   GGA CCT GGT CAG CAG ATC ATT TTG CCA CTC AAA AAA CTG TCT GTT GAA      2127
   Gly Pro Gly Gln Gln Ile Ile Leu Pro Leu Lys Lys Leu Ser Val Glu
   105                 110                 115                 120
   TAT AGT GCC TTA CCT GTC TTA GGT TCG GCA CCT GTT GTT GCT GCA GGT      2175
   Tyr Ser Ala Leu Pro Val Leu Gly Ser Ala Pro Val Val Ala Ala Gly
                   125                 130                 135
   GGT GTC GCT GGT CAT ACG AAT AAA ATG ACT AAA ATG TCC CCG GAC GCG      2223
   Gly Val Ala Gly His Thr Asn Lys Met Thr Lys Met Ser Pro Asp Ala
               140                 145                 150
   ACT AAA AGC AAC ACG ACC GAT GAC AAG GCT CTA AAT TAT GCG GCA CAA      2271
   Thr Lys Ser Asn Thr Thr Asp Asp Lys Ala Leu Asn Tyr Ala Ala Gln
           155                 160                 165
   CAG GCC GCG AGC CTT GGT AGC CAG CTC CAG TCG CGC TCA CTG AAC GGC      2319
   Gln Ala Ala Ser Leu Gly Ser Gln Leu Gln Ser Arg Ser Leu Asn Gly
       170                 175                 180
   GAT TAC GCG AAA GAT ACC GCT CTT GGT ATG GCC AGC AGC CAG GCT TCG      2367
   Asp Tyr Ala Lys Asp Thr Ala Leu Gly Met Ala Ser Ser Gln Ala Ser
   185                 190                 195                 200
   TCA CAG TTG CAG GCC TGG TTA CAA CAT TAT GGA ACG GCA GAG GTT AAT      2415
   Ser Gln Leu Gln Ala Trp Leu Gln His Tyr Gly Thr Ala Glu Val Asn
                   205                 210                 215
   CTG CAG AGT GGT AAT AAC TTT GAC GGT AGT TCA CTG GAC TTC TTA TTA      2463
   Leu Gln Ser Gly Asn Asn Phe Asp Gly Ser Ser Leu Asp Phe Leu Leu
               220                 225                 230
   CCG TTC TAT GAT TCC GAA AAC ATG CTG GCA TTT GGT CAG GTC GGT GCG      2511
   Pro Phe Tyr Asp Ser Glu Asn Met Leu Ala Phe Gly Gln Val Gly Ala
           235                 240                 245
   CGT TAC ATT GAC TCC CGC TTT ACG GCA AAT TTA GGT GCT GGC CAG CGT      2559
   Arg Tyr Ile Asp Ser Arg Phe Thr Ala Asn Leu Gly Ala Gly Gln Arg
       250                 255                 260
   TTT TTC CTT CCT GAA AAT ATG TTG GGC TAT AAC GTC TTC ATT GAT CAG      2607
   Phe Phe Leu Pro Glu Asn Met Leu Gly Tyr Asn Val Phe Ile Asp Gln
   265                 270                 275                 280
   GAT TTT TCT GGT GAT AAT ACC CGT TTA GGT ATT GGT GGC GAA TAC TGG      2655
   Asp Phe Ser Gly Asp Asn Thr Arg Leu Gly Ile Gly Gly Glu Tyr Trp
                   285                 290                 295
   CGA GAC TAT TTC AAA AGT AGC GTT AAC GGC TAT TTC CGC ATG AGC GGC      2703
   Arg Asp Tyr Phe Lys Ser Ser Val Asn Gly Tyr Phe Arg Met Ser Gly
               300                 305                 310
   TGG CAT GAG TCA TAC AAT AAG AAA GAC TAT GAT GAG CGC CCG GCA AAT      2751
   Trp His Glu Ser Tyr Asn Lys Lys Asp Tyr Asp Glu Arg Pro Ala Asn
           315                 320                 325
   GGT TTT GAT ATC CGC TTT AAT GGC TAT TTA CCA TCA TAT CCG GCA TTA      2799
   Gly Phe Asp Ile Arg Phe Asn Gly Tyr Leu Pro Ser Tyr Pro Ala Leu
       330                 335                 340
   GGC GCC AAA CTG ATG TAC GAA CAG TAT TAT GGT GAT AAT GTT GCT TTG      2847
   Gly Ala Lys Leu Met Tyr Glu Gln Tyr Tyr Gly Asp Asn Val Ala Leu
   345                 350                 355                 360
   TTT AAT TCC GAT AAG TTG CAG TCG AAT CCT GGC GCG GCG ACC GTT GGT      2895
   Phe Asn Ser Asp Lys Leu Gln Ser Asn Pro Gly Ala Ala Thr Val Gly
                   365                 370                 375
   GTA AAC TAC ACT CCG ATT CCT CTG GTG ACG ATG GGG ATC GAT TAC CGT      2943
   Val Asn Tyr Thr Pro Ile Pro Leu Val Thr Met Gly Ile Asp Tyr Arg
               380                 385                 390
   CAT GGT ACG GGT AAT GAA AAT GAT CTC CTT TAC TCA ATG CAG TTC CGT      2991
   His Gly Thr Gly Asn Glu Asn Asp Leu Leu Tyr Ser Met Gln Phe Arg
           395                 400                 405
   TAT CAG TTT GAT AAA CCG TGG TCT CAG CAA ATC GAG CCA CAG TAT GTT      3039
   Tyr Gln Phe Asp Lys Pro Trp Ser Gln Gln Ile Glu Pro Gln Tyr Val
       410                 415                 420
   AAC GAG TTA AGA ACA TTA TCG GGC AGC CGT TAC GAT CTG GTT CAG CGT      3087
   Asn Glu Leu Arg Thr Leu Ser Gly Ser Arg Tyr Asp Leu Val Gln Arg
   425                 430                 435                 440
   AAT AAC AAT ATT ATT CTG GAG TAC AAA AAG CAG GAT ATT CTT TCT CTG      3135
   Asn Asn Asn Ile Ile Leu Glu Tyr Lys Lys Gln Asp Ile Leu Ser Leu
                   445                 450                 455
   AAT ATT CCG CAT GAT ATT AAT GGT ACT GAA CAC AGT ACG CAG AAG ATT      3183
   Asn Ile Pro His Asp Ile Asn Gly Thr Glu His Ser Thr Gln Lys Ile
               460                 465                 470
   CAA TTG ATC GTT AAG AGC AAA TAC GGT CTG GAT CGT ATC GTC TGG GAT      3231
   Gln Leu Ile Val Lys Ser Lys Tyr Gly Leu Asp Arg Ile Val Trp Asp
           475                 480                 485
   GAT AGC GCA TTA CGC AGT CAG GGC GGT CAG ATT CAG CAT GGC GGA AGC      3279
   Asp Ser Ala Leu Arg Ser Gln Gly Gly Gln Ile Gln His Gly Gly Ser
       490                 495                 500
   CAA AGC GCA CAA GAC TAC CAG GCT ATT TTG CCT GCT TAT GTG CAA GGC      3327
   Gln Ser Ala Gln Asp Tyr Gln Ala Ile Leu Pro Ala Tyr Val Gln Gly
   505                 510                 515                 520
   GGC AGC AAT ATT TAT AAA GTG ACC GCT CGC GCC TAT GAC CGA AAT GGT      3375
   Gly Ser Asn Ile Tyr Lys Val Thr Ala Arg Ala Tyr Asp Arg Asn Gly
                   525                 530                 535
   AAT AGT TCT AAT AAT GTA CAG CTC ACT ATT ACC GTT TTA CCG AAT GGG      3423
   Asn Ser Ser Asn Asn Val Gln Leu Thr Ile Thr Val Leu Pro Asn Gly
               540                 545                 550
   CAG GTT GTG GAC CAG GTT GGG GTA ACG GAC TTT ACG GCT GAT AAA ACA      3471
   Gln Val Val Asp Gln Val Gly Val Thr Asp Phe Thr Ala Asp Lys Thr
           555                 560                 565
   TCG GCT AAA GCG GAT GGC ATA GAA GCT ATT ACC TAT ACC GCG ACG GTT      3519
   Ser Ala Lys Ala Asp Gly Ile Glu Ala Ile Thr Tyr Thr Ala Thr Val
       570                 575                 580
   AAA AAG AAT GGT GTA GCT CAG GCT AAT GTC CCT GTA ACA TTT AGT ATT      3567
   Lys Lys Asn Gly Val Ala Gln Ala Asn Val Pro Val Thr Phe Ser Ile
   585                 590                 595                 600
   GTA TCC GGG ACT GCA ACT CTT GGG GCA AAT AGT GCC AGA ACG GAT GGT      3615
   Val Ser Gly Thr Ala Thr Leu Gly Ala Asn Ser Ala Arg Thr Asp Gly
                   605                 610                 615
   AAC GGT AAG GCG ACC GTA ACG CTG AAG TCG GCT ACG CCA GGA CAG GTC      3663
   Asn Gly Lys Ala Thr Val Thr Leu Lys Ser Ala Thr Pro Gly Gln Val
               620                 625                 630
   GTC GTG TCT GCT AAA ACC GCG GAG ATG ACT TCG CCA CTT AAT GCC AGC      3711
   Val Val Ser Ala Lys Thr Ala Glu Met Thr Ser Pro Leu Asn Ala Ser
           635                 640                 645
   GCG GTT ATA TTT GTT GAT CAA ACC AAG GCC AGT ATT ACT GAG ATT AAG      3759
   Ala Val Ile Phe Val Asp Gln Thr Lys Ala Ser Ile Thr Glu Ile Lys
       650                 655                 660
   GCT GAT AAA ACA ACA GCG AAG GCA GAT GGT TCT GAT GCG ATT ACC TAT        3807
   Ala Asp Lys Thr Thr Ala Lys Ala Asp Gly Ser Asp Ala Ile Thr Tyr
   665                 670                 675                 680
   ACT GTC AGA GTG ATG AAG GAG GGG GCA CCC GTA GTA GAT CAG AAA GTG        3855
   Thr Val Arg Val Met Lys Glu Gly Ala Pro Val Val Asp Gln Lys Val
                   685                 690                 695
   ACC TTT TCT AAG GAT TTT GGG ACC CTG AAT AAG ACT GAA GCA ACA ACC        3903
   Thr Phe Ser Lys Asp Phe Gly Thr Leu Asn Lys Thr Glu Ala Thr Thr
               700                 705                 710
   GAT CAG AAT GGT TAT GCT ACT GTA AAA TTA TCA TCC AAT ACT CCT GGC        3951
   Asp Gln Asn Gly Tyr Ala Thr Val Lys Leu Ser Ser Asn Thr Pro Gly
           715                 720                 725
   AAG GCC ATT GTT AGT GCA AAA GTG AGT GGA GTA GGT ACA GAA GTT AAG        3999
   Lys Ala Ile Val Ser Ala Lys Val Ser Gly Val Gly Thr Glu Val Lys
       730                 735                 740
   GCT ACT ACC GTT GAG TTT TTT GCC CCG TTG AGT ATT GAT GGT GAT AAA        4047
   Ala Thr Thr Val Glu Phe Phe Ala Pro Leu Ser Ile Asp Gly Asp Lys
   745                 750                 755                 760
   GTG ACC GTA ATT GGT ACT GGT ATC ACG GGG GCT CTG CCA AAG AAC TGG        4095
   Val Thr Val Ile Gly Thr Gly Ile Thr Gly Ala Leu Pro Lys Asn Trp
                   765                 770                 775
   TTA CAG TAT GGT CAG GTT AAG CTA CAG GCA ACA GGG GGC AAT GGA AAA        4143
   Leu Gln Tyr Gly Gln Val Lys Leu Gln Ala Thr Gly Gly Asn Gly Lys
               780                 785                 790
   TAC ACA TGG AAA TCC AGT AAT ACT AAA ATT GCT TCT GTT GAT AAC TCG        4191
   Tyr Thr Trp Lys Ser Ser Asn Thr Lys Ile Ala Ser Val Asp Asn Ser
           795                 800                 805
   GGA GTG ATA ACC TTA AAT GAA AAA GGG AGT GCC ACA ATT ACT GTA GTA        4239
   Gly Val Ile Thr Leu Asn Glu Lys Gly Ser Ala Thr Ile Thr Val Val
       810                 815                 820
   TCT GGT GAT AAT CAG AGT GCG ACA TAC ACA ATT AAT GCA CCG GGT AGT        4287
   Ser Gly Asp Asn Gln Ser Ala Thr Tyr Thr Ile Asn Ala Pro Gly Ser
   825                 830                 835                 840
   ATT GTA ATT GCT GTG GAT AAA AAT ACT CGA GTT ACG TAT TTT GAT GCC        4335
   Ile Val Ile Ala Val Asp Lys Asn Thr Arg Val Thr Tyr Phe Asp Ala
                   845                 850                 855
   GAA AAC AAA TGT AAG ACA AAT AGC GCA AAT TTA GCA CAG CCA AAA GAA        4383
   Glu Asn Lys Cys Lys Thr Asn Ser Ala Asn Leu Ala Gln Pro Lys Glu
               860                 865                 870
   CTA TTG GCC AAT ATC TAT TCA ACA TGG GGT GCT GCA AAT AAA TAT CCT        4431
   Leu Leu Ala Asn Ile Tyr Ser Thr Trp Gly Ala Ala Asn Lys Tyr Pro
           875                 880                 885
   TAC TAT TCT GGT TCT AAA TCA TTG ACT GCT TGG ATT AAA CAA TCT TCT     4479
   Tyr Tyr Ser Gly Ser Lys Ser Leu Thr Ala Trp Ile Lys Gln Ser Ser
       890                 895                 900
   TCT GAA CAG TCA TCA GGT GTA TCA AGC ACA TAT GAT TTG GTT ACG AAG     4527
   Ser Glu Gln Ser Ser Gly Val Ser Ser Thr Tyr Asp Leu Val Thr Lys
   905                 910                 915                 920
   AAC CAG TTG ATC AAT GTT GGA GTA AAC AAT AAG AAT GCT TTT TCT GTT     4575
   Asn Gln Leu Ile Asn Val Gly Val Asn Asn Lys Asn Ala Phe Ser Val
                   925                 930                 935
   TGT GTA AAA TAA TTTAATTACA TCTCAAATCC CGGAAAATAA AATTCATTTT         4627
   Cys Val Lys  *
               940
   CCGGGATTTT TTATTGTACC TACTTGAATC TTTTAATAAA CGTTTCATGT TAAAATCAAT   4687
   GTCTCATTTC GGAATAAGTT TTTCGTGTAT CTTGTTTGAT AGAGACCTTG TTTACCATAT   4747
   CTACAACATC TGCACAATAA AAAACCCTCC GAAGAGGGGG AAGATAATAA TAAGTTAATA   4807
   AGACAACGGA ATATGTATAT ATTCGTCTGG GCGGGAGATG TTGATACCAT CTTTAGGATC   4867
   AATCGCGTCT ATGACATAGC CTCCTTTCAG AATGCTACCA ATTCCCAGGC TTGTACCGTT   4927
   ATCAAGAGCA ATTGTTGGAT TCTTCATCGT GCCGCTGATT CTGAGTATTT TTCCGGGGAA   4987
   AATATCTGCT GAAATGTACG GCTGAATGTT CTGAAATAGT ATCTTGACGT TACTATTTTT   5047
   GTTCATCGCA TTTATAATGG CAAGGATCTT ACTCTCGTTC TGTTGAGAAA CCTCCCCCGC   5107
   AACAATGATG ACATCATTCT TTTTGAAAAT ATTGACATAG TTTATTAATT TATTCTTTTT   5167
   GAACTCTGAT GCCAGTTCAT TGATTTTATT GTTACTGTTG TTATGTATTT CCCATCCAGC   5227
   AACCCCTGGC GTATTCAGTA ACAGATTTTC AACTTCGCTC CATTTTGGTG AAGGGGGAAT   5287
   GTATCCCGAT AACAGGAAGA ATCCAGGCTT ATTACCTTTT GTAATGATGA TATCCTTATA   5347
   ACCGTATTCC GTTAAAATAT CATTAATCGC GGAGATGATT TGGTTATTGC AAATGATATT   5407
   GTTGTAATAA AAAATATTTT TTTCTTTCAG GTAATTAAAA AATGGAGTTA AATCAGTGGT   5467
   TGATTCGCAA CGCCCATAAA GCGCGACATT TTTCCCATGC CATACCAGAG TGATTGCATG   5527
   AAGTTTGTTT TCTTTTAGCT GCTGGTATAC ATCAGCTCTG GTAAACGCTG GGGGTTCAGC   5587
   TTTTGGGGAA AGCAAAACGT AACTGCCGAT GGCGCCAGAC AACGCAATCG CAGCACCAAT   5647
   CACGCTAGCC AGGATAAGAC GCTTTTTACG TCGATTTTTG GCAGAGAACC TTTCTGGCAC   5707
   ATCAGACAAT GGCAAGGTGT GGTCAATATC ACCAATGATG AAGCAACATC CCGCTATATC   5767
   GATGACCTTT CCGACAGGTA ATGACTGATC TGCTTCATAG GGGGTGGGCT GTCCATCAAT   5827
   CCAAAATTCA ACCGGAGATA AAAGGAAAAC ACCTTCCTCA CGTATATCAA GTAAAAACTG   5887
   ATTGAGGCCT TGTTCAAGGG GAAAATAAAC AATATCGTTA TCTTCTGTGC CTATCGATAG    5947
   GTTTCCCATC GGAAGTTGTA ATTCCCGATT ACGCATCGCA CCATTGAGAA GTTTTATTTT    6007
   ATATGAGGAT AACATTTTTA AATTTTTTTC ATCCTGGTGG TTGAACAATG AGAAAAAATA    6067
   TGGTGAACTT ACATCAACTA AGAATAGAGT AGAAAGGAAG CTGTTGTACA GTACTATTTA    6127
   ATGGAATATT CATAATTAAT GATTACGTGA GTTTCCAATG GCTAATGGTA TTGAATTTAA    6187
   TCAAAACACC GCATCTGTTT TTAATTCTAA TTCATTAGAT TTTGAATTGG AATCTCAACA    6247
   ATTAACGCAA AAAAATTCTT CTAATAGTTA TTCTCCATTA TTAAATTTAC AAAATGAATT    6307
   AGCGATGATT ACTAGTTCAT CGCTTTCTGA AACGATTGAA GGGCTAAGCC TGGGATATCG    6367
   CAAAGGCAGT GCAAGGAAAG AAGAGGAAGG TACGACAACG GAGAAGCTGC TTAATGATAT    6427
   GCAAGAGCTG CTTACTCTGA CCGATAGCGA TAAATTAAAA GAACTATCAT TAAAGAACAG    6487
   TGGGCTGCTG GAACAGCATG ATCCTAATTT GGCTATGTTT GGTAATATGC CAAAAGGGGA    6547
   GATTGTTGCA CTTATATCTT CTTTATTACA ATCTAAGTTT GTTAAGATTG AATTAAAAAA    6607
   GAAATACGCC AAATTATTAT TAGATTTATT AGGCGAAGAT GATTGGGAGT TGGCTCTGCT    6667
   TTCCTGGTTA GGGGTGGGTG AGTTAAATCA GGAAGGTATC CAGAAGATCA AAAAGCTTTA    6727
   TGAAAAGGCC AAGGATGAGG ATTCTGAAAA GGGAGCCTCT TTATTTGACT GGTTTATGGA    6787
   GATTAAGGAT CTTCCAGAGC GTGAGAAGCA CTTAAAGGTT ATTATTAGGG CGCTATCGTT    6847
   CGATCTGTCT TATATGTCTT CTTTTGAAGA TAAGGTGAAA ACATCTTCAA TTATTAGCGA    6907
   TTTATGCCGG GTAATCATTT TTTTATCGCT TGATAACTAT ACAGATATTA TTGCAATCTC    6967
   TATTAATAAA GATAAAGATG TCATTTTAAA TGAAGTATTG TCTATTATTG AACATGTATG    7027
   GTTAACAGAA GACTGGCTCC TGGAAAGCCC TTCTCGGGTA TCGATTGTCG AAGATAAACA    7087
   TGTTTATTAT TTCCACCTAT TGAAAGACTT TTTTGCATCA TTACCTGACG CTTGTTTTAT    7147
   TGATAGGGAA CAGAGAGATA ATACATTATT AATGATTGGT AAAGTAATTG ATTATAAGGA    7207
   GGATGTTATT TGATATTGGT TTTTTAATCG TTTTTGGTCT TGCTAAGAAA GATTATTAAG    7267
   AGGTATATAC ATG GAT ACA TCA ACT GCA ACA TCA GTT GCT AGT GCG AAC       7316
              Met Asp Thr Ser Thr Ala Thr Ser Val Ala Ser Ala Asn
               1               5                   10
   GCG AGT ACT TCG ACA TCG ACA GTC TAT GAC TTA GGC AGT ATG TCG AAA      7364
   Ala Ser Thr Ser Thr Ser Thr Val Tyr Asp Leu Gly Ser Met Ser Lys
        15                  20                  25
   GAC GAA GTA GTT CAG CTA TTT AAT AAA GTC GGT GTT TTT CAG GCT GCG      7412
   Asp Glu Val Val Gln Leu Phe Asn Lys Val Gly Val Phe Gln Ala Ala
    30                  35                  40                  45
   CTT CTC ATG TTT GCC TAT ATG TAT CAG GCA CAA AGC GAT CTG TCG ATT      7460
   Leu Leu Met Phe Ala Tyr Met Tyr Gln Ala Gln Ser Asp Leu Ser Ile
                    50                  55                  60
   GCA AAG TTT GCT GAT ATG AAT GAG GCA TCT AAG GAG TCA ACC ACA GCC      7508
   Ala Lys Phe Ala Asp Met Asn Glu Ala Ser Lys Glu Ser Thr Thr Ala
                65                  70                  75
   CAA AAA ATG GCT AAT CTT GTG GAT GCT AAA ATT GCT GAT GTT CAG AGT      7556
   Gln Lys Met Ala Asn Leu Val Asp Ala Lys Ile Ala Asp Val Gln Ser
            80                  85                  90
   AGT TCT GAC AAG AAT AAG AAA GCC AAA CTT CCT CAA GAA GTG ATT GAC      7604
   Ser Ser Asp Lys Asn Lys Lys Ala Lys Leu Pro Gln Glu Val Ile Asp
        95                 100                 105
   TAT ATA AAT GAT CCT CGC AAT GAC ATT ACA GTA AGT GGT ATT AGC GAT      7652
   Tyr Ile Asn Asp Pro Arg Asn Asp Ile Thr Val Ser Gly Ile Ser Asp
   110                 115                 120                 125
   CTA AAT GCT GAA TTA GGC GCT GGT GAT TTG CAA ACG GTG AAG GCC GCT      7700
   Leu Asn Ala Glu Leu Gly Ala Gly Asp Leu Gln Thr Val Lys Ala Ala
                   130                 135                 140
   ATT TCG GCC AAA TCG AAT AAC TTG ACC ACG GTA GTG AAT AAT AGC CAG      7748
   Ile Ser Ala Lys Ser Asn Asn Leu Thr Thr Val Val Asn Asn Ser Gln
               145                 150                 155
   CTT GAA ATA CAG CAA ATG TCA AAT ACG TTA AAC CTA TTA ACG AGT GCA      7796
   Leu Glu Ile Gln Gln Met Ser Asn Thr Leu Asn Leu Leu Thr Ser Ala
           160                 165                 170
   CGT TCT GAT ATT CAG TCA CTG CAA TAC AGA ACT ATT TCA GCA ATA TCC      7844
   Arg Ser Asp Ile Gln Ser Leu Gln Tyr Arg Thr Ile Ser Ala Ile Ser
       175                 180                 185
   CTT GGT AAA TAA CCGGAGATAA CT ATG CTT AAT GTA AAT AGC GAT ATC        7892
   Leu Gly Lys  *                Met Leu Asn Val Asn Ser Asp Ile
   190                             1               5
   CAG TCT ATG AGG TCT GGG GCC AGC GCT GCT ACG GCT ACT TCA GGT ATT      7940
   Gln Ser Met Arg Ser Gly Ala Ser Ala Ala Thr Ala Thr Ser Gly Ile
        10                  15                  20
   AAT CAG CCT GAA GTG ACT TCC GCG CTG GAT TTA CAA CTG GTT AAA TCC      7988
   Asn Gln Pro Glu Val Thr Ser Ala Leu Asp Leu Gln Leu Val Lys Ser
    25                  30                  35                  40
   ACG GCC CCA TCA GCA AGT TGG ACA GAA AGT ACC GCT TTA CCA ACG CCA      8036
   Thr Ala Pro Ser Ala Ser Trp Thr Glu Ser Thr Ala Leu Pro Thr Pro
                    45                  50                  55
   CCG GCA GGC CAC TCA TTA GTG ACG CCC TCT GTT GCT GAG GAT GTC CTG      8084
   Pro Ala Gly His Ser Leu Val Thr Pro Ser Val Ala Glu Asp Val Leu
                60                  65                  70
   AGT AAA CTG TTT GGT GGT ATT AGT GGT GAA GTT AGG AGT CGA ACA GAG      8132
   Ser Lys Leu Phe Gly Gly Ile Ser Gly Glu Val Arg Ser Arg Thr Glu
            75                  80                  85
   GGA ACC GAA CCA CAG CGC AGC ACT CAG AAT GCA AGC TCT GGC TAT CCT      8180
   Gly Thr Glu Pro Gln Arg Ser Thr Gln Asn Ala Ser Ser Gly Tyr Pro
        90                  95                 100
   TAT CTT TCT CAG GTG AAT AAC GTT GAC CCT CAG GCC ATG ATG ATG ATG      8228
   Tyr Leu Ser Gln Val Asn Asn Val Asp Pro Gln Ala Met Met Met Met
   105                 110                 115                 120
   GTC ACC CTG TTA TCC CTG GAT GCT TCA GCG CAG AAA GTT GCG AGT ATG      8276
   Val Thr Leu Leu Ser Leu Asp Ala Ser Ala Gln Lys Val Ala Ser Met
                   125                 130                 135
   AAA AAC TCC AAC GAG ATC TAC ACG GAT GGG CAA AAT AAG GCG CTG GAT      8324
   Lys Asn Ser Asn Glu Ile Tyr Thr Asp Gly Gln Asn Lys Ala Leu Asp
               140                 145                 150
   AAT AAA ACG CTG GAG TTT AAA AAG CAG CTC GAA GAA CAA CAG AAA GCC      8372
   Asn Lys Thr Leu Glu Phe Lys Lys Gln Leu Glu Glu Gln Gln Lys Ala
           155                 160                 165
   GAA GAG AAA GCA CAA AAA AGT AAA ATT CTT GGT CAG GTC TTT GGT TGG      8420
   Glu Glu Lys Ala Gln Lys Ser Lys Ile Leu Gly Gln Val Phe Gly Trp
       170                 175                 180
   TTG GGC GTC GCG GTA ACT GCT ATT GCA GCT ATT TTT AAC CCG GCA CTC      8468
   Leu Gly Val Ala Val Thr Ala Ile Ala Ala Ile Phe Asn Pro Ala Leu
   185                 190                 195                 200
   TGG GCT GTT GTT GCC GTT AGC GCA ACA GCA ATG GCA CTG CAA ACT GCA      8516
   Trp Ala Val Val Ala Val Ser Ala Thr Ala Met Ala Leu Gln Thr Ala
                   205                 210                 215
   GTT GAT GTA ATG GGC GAT AAG GCC CCG CAG GCG TTA AAG ACA GCA GCG      8564
   Val Asp Val Met Gly Asp Lys Ala Pro Gln Ala Leu Lys Thr Ala Ala
               220                 225                 230
   CAA GTC TTT GGC GGA GTA TCT ATT GCT GCA AGT ATT CTG ACA GCC GGC      8612
   Gln Val Phe Gly Gly Val Ser Ile Ala Ala Ser Ile Leu Thr Ala Gly
           235                 240                 245
   ATT GGC GGG GTG TCT TCA CTT ATG TCT AAA GTT GGC GAT GTC GCT AAC      8660
   Ile Gly Gly Val Ser Ser Leu Met Ser Lys Val Gly Asp Val Ala Asn
       250                 255                 260
   AAA GTT GGT TCA AAC ATC GTG AAA ACA GTT ACT AAA CTG GCC GAT GTA      8708
   Lys Val Gly Ser Asn Ile Val Lys Thr Val Thr Lys Leu Ala Asp Val
   265                 270                 275                 280
   TTT GTT GAG AAC GTT GTT TCA AAA GTT GCG GCA ACG GCA AAT GGA TTT      8756
   Phe Val Glu Asn Val Val Ser Lys Val Ala Ala Thr Ala Asn Gly Phe
                   285                 290                 295
   ACG ACC TCT GCA CGT TCT ATT GGG ACA ACT GTG CTG AAT AAT GAT TCT       8804
   Thr Thr Ser Ala Arg Ser Ile Gly Thr Thr Val Leu Asn Asn Asp Ser
               300                 305                 310
   GCG CAA TAC AGC ATA TTG GCT CAG TTA TCT GCT TAC GCG GTA CAA AAC       8852
   Ala Gln Tyr Ser Ile Leu Ala Gln Leu Ser Ala Tyr Ala Val Gln Asn
           315                 320                 325
   TTA ACT CGA CAG AGT GAA AAC CTG GGT GAG AGT GCG AAG GTT GAG CTG       8900
   Leu Thr Arg Gln Ser Glu Asn Leu Gly Glu Ser Ala Lys Val Glu Leu
       330                 335                 340
   GAT AAA GCT GCA GCC GAG TTG CGA AAC CAG GCA AGC TAT TTA CAA AAT       8948
   Asp Lys Ala Ala Ala Glu Leu Arg Asn Gln Ala Ser Tyr Leu Gln Asn
   345                 350                 355                 360
   GCT TCT CAG TTA ATA TCC GAC TCA GCA CGA GTA AAT AGC CGT ATT GTT       8996
   Ala Ser Gln Leu Ile Ser Asp Ser Ala Arg Val Asn Ser Arg Ile Val
                   365                 370                 375
   AGT GGT CGA GTT TAA TTATTAAAGA GAATTTAATT ATG AAT ACT ATT GAT         9046
   Ser Gly Arg Val  *                         Met Asn Thr Ile Asp
               380                             1               5
   TAT ACT AAT CAA GTA ATG ACG GTT AAT TCT GTT TCG GAG AAT ACT ACC       9094
   Tyr Thr Asn Gln Val Met Thr Val Asn Ser Val Ser Glu Asn Thr Thr
                   10                  15                  20
   GGC TCT AAT GCA ATT ACC GCA TCT GCT ATT AAT TCA TCT TTG CTT ACC       9142
   Gly Ser Asn Ala Ile Thr Ala Ser Ala Ile Asn Ser Ser Leu Leu Thr
               25                  30                  35
   GAT GGT AAG GTC GAT GTT TCT AAA CTG ATG CTG GAA ATT CAA AAA CTC       9190
   Asp Gly Lys Val Asp Val Ser Lys Leu Met Leu Glu Ile Gln Lys Leu
           40                  45                  50
   CTG GGC AAG ATG GTG CGT ATA TTG CAG GAT TAC CAA CAG CAA CAG TTG       9238
   Leu Gly Lys Met Val Arg Ile Leu Gln Asp Tyr Gln Gln Gln Gln Leu
       55                  60                  65
   TCG CAG AGC TAT CAG ATC CAA CTG GCC GTT TTT GAG AGC CAG AAT AAA       9286
   Ser Gln Ser Tyr Gln Ile Gln Leu Ala Val Phe Glu Ser Gln Asn Lys
   70                  75                  80                  85
   GCC ATT GAT GAA AAA AAG GCC GCT GCA ACA GCC GCT CTG GTT GGT GGG       9334
   Ala Ile Asp Glu Lys Lys Ala Ala Ala Thr Ala Ala Leu Val Gly Gly
                   90                  95                  100
   GCT ATT TCA TCA GTA TTG GGG ATC TTA GGC TCT TTT GCA GCA ATT AAC       9382
   Ala Ile Ser Ser Val Leu Gly Ile Leu Gly Ser Phe Ala Ala Ile Asn
               105                 110                 115
   AGT GCT ACG AAA GGC GCG AGT GAT ATT GCT CAA AAA ACC GCC TCT ACA       9430
   Ser Ala Thr Lys Gly Ala Ser Asp Ile Ala Gln Lys Thr Ala Ser Thr
           120                 125                 130
   TCT TCT AAG GCT ATT GAT GCG GCT TCT GAT ACT GCG ACT AAA ACG TTG         9478
   Ser Ser Lys Ala Ile Asp Ala Ala Ser Asp Thr Ala Thr Lys Thr Leu
       135                 140                 145
   ACT AAG GCA ACG GAA AGC GTT GCT GAT GCT GTT GAA GAT GCA TCC AGC         9526
   Thr Lys Ala Thr Glu Ser Val Ala Asp Ala Val Glu Asp Ala Ser Ser
   150                 155                 160                 165
   GTG ATG CAG CAA GCG ATG ACT ACA GCA ACG AGA GCG GCC AGC CGT ACA         9574
   Val Met Gln Gln Ala Met Thr Thr Ala Thr Arg Ala Ala Ser Arg Thr
                   170                 175                 180
   TCC GAC GTT GCT GAT GAC ATT GCC GAT TCT GCT CAG AGA GCT TCT CAG         9622
   Ser Asp Val Ala Asp Asp Ile Ala Asp Ser Ala Gln Arg Ala Ser Gln
               185                 190                 195
   CTG GCT GAA AAC GCT GCA GAT GCC GCT CAG AAG GCA AGT CGG GCA AGC         9670
   Leu Ala Glu Asn Ala Ala Asp Ala Ala Gln Lys Ala Ser Arg Ala Ser
           200                 205                 210
   CGC TTT ATG GCT GCA GTA GAT AAG ATT ACT GGC TCT ACA CCA TTT ATT         9718
   Arg Phe Met Ala Ala Val Asp Lys Ile Thr Gly Ser Thr Pro Phe Ile
       215                 220                 225
   GCC GTT ACC AGT CTT GCC GAA GGC ACG AAG ACA TTG CCA ACA ACG GTA         9766
   Ala Val Thr Ser Leu Ala Glu Gly Thr Lys Thr Leu Pro Thr Thr Val
   230                 235                 240                 245
   TCT GAA TCA GTC AAA TCT AAC CAT GAG ATT AGC GAA CAG CGT TAT AAG         9814
   Ser Glu Ser Val Lys Ser Asn His Glu Ile Ser Glu Gln Arg Tyr Lys
                   250                 255                 260
   TCT GTG GAG AAC TTC CAG CAG GGT AAT TTG GAT CTG TAT AAG CAA GAA         9862
   Ser Val Glu Asn Phe Gln Gln Gly Asn Leu Asp Leu Tyr Lys Gln Glu
               265                 270                 275
   GTT CGC AGA GCG CAG GAT GAT ATC GCT AGC CGT CTG CGT GAT ATG ACA         9910
   Val Arg Arg Ala Gln Asp Asp Ile Ala Ser Arg Leu Arg Asp Met Thr
           280                 285                 290
   ACA GCC GCT CGC GAT CTC ACT GAT CTT CAG AAT CGT ATG GGT CAA TCG         9958
   Thr Ala Ala Arg Asp Leu Thr Asp Leu Gln Asn Arg Met Gly Gln Ser
       295                 300                 305
   GTT CGC TTA GCT GGG TAA TTGATCATGG TCGATACGTT TAATGATGAA               10006
   Val Arg Leu Ala Gly  *
   310                 315
   GTGCTTAATC ACTATCTTGA ACAAAAAGGG TACACAATAC AGAAGGAGTT TCTTTGTGGC   10066
   AGTGCCTTTT TTATCGGATG GCGGATTGAG ACCTCTTTTT TCTCATTAGC GTACAGACTT   10126
   GATGAACAAG AACTGATTTT GTGCTCTTTT GAAGCACGTA ACCAAACAGG GCTTAACGGC   10186
   CCTGTTTTAT CACTGACTCG CTTGCTCGAA GAGTTGTACC ACCATTTTTC GGGTATTAAG   10246
   AAAATCAGTG CGATGAAATC TAAGATTGGT TCAGATTCAG AACGTCAAAA GCGCGAAGAG   10306
   TTGTTTAATT ACTTCATCAG AAAGGGCGCT GTGCAGCAAG AAACAGAAGA CGGAATTTGG 10366
   TTCGTAATGA ATGTAAATAG TTAGAAATTT TTATTTCTAT TAACTATTGA GGAAAATTTA 10426
   ATGAATTTAT CTGAAATTAC TCAACAAATG GGTGAAGTAG GTAAAACGCT GAGTGATTCT 10486
   GTGCCAGAGT TACTTAATAG CACCGATTTG GTTAATGACC CTGAAAAAAT GTTAGAGCTG 10546
   CAGTTCGCGG TTCAGCAATA TTCTGCTTAT GTTAACGTAG AAAGTGGAAT GCTGAAAACG 10606
   ATAAAAGATT TGGTCTCAAC CATTTCCAAC CGTAGCTTTT AATTTTTATG GTTAATGATG 10666
   TTTCTGCCAA CAAGCTACTG GTGTGGGCGG CTGTAGCTGC GGCAAATCAT AAGCTTCCCA 10726
   AATATGCAGA ATCTATCCTG GATGTATTGC CGCAAATTAT ACCCGATAAA AAAGATATCG 10786
   CACATTTAGA ATTTATTATT TTATATGGAT TAAATAGAAA AAATGATGCG GTAAAGGCTC 10846
   TGGAGGACTT TATGGATGAT GAGACAAGCC AGTTGTTATG CTGTCTGGTC CACGAGAATA 10906
   ATAGAAGCTG GACACTAATG TTTTAATCTC ATATTGAGTT TAAACAATTG ATAAATTTCA 10966
   TTTTGGTATG CTATAAATTT TAACAGAGAT ATTTATAATA CCTTAATGAA AAATATAGCT 11026
   GTACGAAAGT TTCTATGTTT CCATGTGTGT TTGATTTAAT AGTTTATAGT CGTTAACTAT 11086
   TTTTGATTTT AATATAAATT AATAATTATT CCTGTTAATT AGATATAAAA AGGCATGAAT 11146
   TATGCTTAAT GGAATTAGTC AAGCTGTTTC TACACTAGGA CGGCATATTA CTAGTGCGGC 11206
   AAGCCGGGTA AGCTCTGCGG GGTTATCTGG CTTTTCAGTG TCCCCTCAGG CTGTTCGTCT 11266
   GAATCCGATT AGGGCTCAAT CTCCTTTTTC TCCCGGTACG TCGAATATCA ATGCGAGAAC 11326
   GACTTTTAAT GTGAGTAGCC CGGCGACTTC ATTTACTCCC TCTCGTCCGG CCCCACCGCC 11386
   GCCGACAAGT GGACAGGCTT CCGGGGCATC CCGCCCTTTA CCGCCCATTG CTCAGGCATT 11446
   AAAAGATCAT TTAGCTGCCT ATGAAAAGTC GAAAAGTCTT GATACTTCAA GCCTTAAGCC 11506
   AAGCCGCCCG GCACCGCCGC CGCCGACAAG CGGGTTGGTA TCTGGGTCAT CCCGGTCTTT 11566
   ACCGCCCATT GCTCAGGCGC TAAAAGATCA TTTGGCTGCC TATGAAAAAT CGAAAAGTCT 11626
   TGATACTTCA GGTCTTAAGC CAAGCCGCCC GGCACCGCCG CCACCGACAA GTGGACAGGC 11686
   ATCCGAGGCA TCCCGGCCAT TACCGCCTAT TGCTCAGGCA TTAAAAGATC ATTTAGCTGC 11746
   CTATGAACTG TCGAAAAAGG CATAAAAAGT AGATTTAAAA TTATTAATTA CTGTTGATTG 11806
   TTTAGAGAGT TAAAAGATAA AAACCGATAT TAAGTTGCAT TGTGATAAAT GTGATTGGTA 11866
   ATATCGGTTT TTATTGAAAT AAATATTGTA ATAAATGATG AAAGAGGGTA ATAGAGCAAG 11926
   AGGGGGGTGT TTCGTTAACC AGAGCCTGTA ATTTTTTATC CATAAATTTA CCGCTATTTG 11986
   ATGTTGGATT TGACATATCA ACATCAGGCA ACGACACAAC TCTATGTACA GGCTCTTATA 12046
   GGTATTTTTT TTCTGGTTAG CGTTAAGGGA TATCTCTATT TATGCCGATA TGACGCTGGC    12106
   AGCCATAATA TATCTGGGGA TGTCATCTCG TAGCTGTTTG ATGTCAACAT CTGGACTAAT    12166
   ATGCTTTTGG AAGAACTGGT CTGCGAGTTG ATTGGCTATT ACATCCATTG TCTGGTTCTT    12226
   TACTTCTCTA CTCACTCCCT TGCAAAGCAT ATTTATTTTA TCACCGAAAC GACCATTTCC    12286
   TATTATGAGA TGGTTGATAG AGCTTTGCGC TGCTATGATA TTCAACTTGA GACTGTAATC    12346
   CTTTTCAATG AGTTTAAAAA CTCTTTGCCG TACTTCACTG GAAATGTTTC TGCTATTTTC    12406
   AGCACAATAT TTGTCAATTC CCTGGTTGAT CACGCGAGAT GGGTAAACTG TTCGCTCATC    12466
   TCTTTTCCAG GCATTAATAT CTTTGGTACT TTGTCGGGCG ACGGAGGTCA AAATTCGGTT    12526
   GTTCAGCGTA TTTGTGGATA ACTTGATAGT CCTCTGACCC ATTGAACATC GCAGGATATC    12586
   ATCAGATTTC CTCCAGCAGG ACTGTTTCTG GATAAAGCTT                          12626
(2) INFORMATIONS POUR LA SEQ ID NO: 2:
   (i) CARACTERISTIQUES DE LA SEQUENCE:
      (A) LONGUEUR: 940 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 2:
   Met Ile Thr His Gly Phe Tyr Ala Arg Thr Arg His Lys His Lys Leu
    1               5                  10                  15
   Lys Lys Thr Phe Ile Met Leu Ser Ala Gly Leu Gly Leu Phe Phe Tyr
               20                  25                  30
   Val Asn Gln Asn Ser Phe Ala Asn Gly Glu Asn Tyr Phe Lys Leu Ser
           35                  40                  45
   Ser Asp Ser Lys Leu Leu Thr Gln Asn Ala Ala Gln Asp Arg Leu Phe
       50                  55                  60
   Tyr Thr Leu Lys Thr Gly Glu Thr Val Ala Asn Ile Ser Lys Ser Gln
   65                  70                  75                  80
   Gly Ile Ser Leu Ser Val Ile Trp Ser Leu Asn Lys His Leu Tyr Ser
                   85                  90                  95
   Ser Glu Ser Glu Met Met Lys Ala Gly Pro Gly Gln Gln Ile Ile Leu
               100                 105                 110
   Pro Leu Lys Lys Leu Ser Val Glu Tyr Ser Ala Leu Pro Val Leu Gly
           115                 120                 125
   Ser Ala Pro Val Val Ala Ala Gly Gly Val Ala Gly His Thr Asn Lys
       130                 135                 140
   Met Thr Lys Met Ser Pro Asp Ala Thr Lys Ser Asn Thr Thr Asp Asp
   145                 150                 155                 160
   Lys Ala Leu Asn Tyr Ala Ala Gln Gln Ala Ala Ser Leu Gly Ser Gln
                   165                 170                 175
   Leu Gln Ser Arg Ser Leu Asn Gly Asp Tyr Ala Lys Asp Thr Ala Leu
               180                 185                 190
   Gly Met Ala Ser Ser Gln Ala Ser Ser Gln Leu Gln Ala Trp Leu Gln
           195                 200                 205
   His Tyr Gly Thr Ala Glu Val Asn Leu Gln Ser Gly Asn Asn Phe Asp
       210                 215                 220
   Gly Ser Ser Leu Asp Phe Leu Leu Pro Phe Tyr Asp Ser Glu Asn Met
   225                 230                 235                 240
   Leu Ala Phe Gly Gln Val Gly Ala Arg Tyr Ile Asp Ser Arg Phe Thr
                   245                 250                 255
   Ala Asn Leu Gly Ala Gly Gln Arg Phe Phe Leu Pro Glu Asn Met Leu
               260                 265                 270
   Gly Tyr Asn Val Phe Ile Asp Gln Asp Phe Ser Gly Asp Asn Thr Arg
           275                 280                 285
   Leu Gly Ile Gly Gly Glu Tyr Trp Arg Asp Tyr Phe Lys Ser Ser Val
       290                 295                 300
   Asn Gly Tyr Phe Arg Met Ser Gly Trp His Glu Ser Tyr Asn Lys Lys
   305                 310                 315                 320
   Asp Tyr Asp Glu Arg Pro Ala Asn Gly Phe Asp Ile Arg Phe Asn Gly
                   325                 330                 335
   Tyr Leu Pro Ser Tyr Pro Ala Leu Gly Ala Lys Leu Met Tyr Glu Gln
               340                 345                 350
   Tyr Tyr Gly Asp Asn Val Ala Leu Phe Asn Ser Asp Lys Leu Gln Ser
           355                 360                 365
   Asn Pro Gly Ala Ala Thr Val Gly Val Asn Tyr Thr Pro Ile Pro Leu
       370                 375                 380
   Val Thr Met Gly Ile Asp Tyr Arg His Gly Thr Gly Asn Glu Asn Asp
   385                 390                 395                 400
   Leu Leu Tyr Ser Met Gln Phe Arg Tyr Gln Phe Asp Lys Pro Trp Ser
                   405                 410                 415
   Gln Gln Ile Glu Pro Gln Tyr Val Asn Glu Leu Arg Thr Leu Ser Gly
               420                 425                 430
   Ser Arg Tyr Asp Leu Val Gln Arg Asn Asn Asn Ile Ile Leu Glu Tyr
           435                 440                 445
   Lys Lys Gln Asp Ile Leu Ser Leu Asn Ile Pro His Asp Ile Asn Gly
   450 455 460
   Thr Glu His Ser Thr Gln Lys Ile Gln Leu Ile Val Lys Ser Lys Tyr
   465 470 475 480
   Gly Leu Asp Arg Ile Val Trp Asp Asp Ser Ala Leu Arg Ser Gln Gly
   485 490 495
   Gly Gln Ile Gln His Gly Gly Ser Gln Ser Ala Gln Asp Tyr Gln Ala
   500 505 510
   Ile Leu Pro Ala Tyr Val Gln Gly Gly Ser Asn Ile Tyr Lys Val Thr
   515 520 525
   Ala Arg Ala Tyr Asp Arg Asn Gly Asn Ser Ser Asn Asn Val Gln Leu
   530 535 540
   Thr Ile Thr Val Leu Pro Asn Gly Gln Val Val Asp Gln Val Gly Val
   545 550 555 560
   Thr Asp Phe Thr Ala Asp Lys Thr Ser Ala Lys Ala Asp Gly Ile Glu
   565 570 575
   Ala Ile Thr Tyr Thr Ala Thr Val Lys Lys Asn Gly Val Ala Gln Ala
   580 585 590
   Asn Val Pro Val Thr Phe Ser Ile Val Ser Gly Thr Ala Thr Leu Gly
   595 600 605
   Ala Asn Ser Ala Arg Thr Asp Gly Asn Gly Lys Ala Thr Val Thr Leu
   610 615 620
   Lys Ser Ala Thr Pro Gly Gln Val Val Val Ser Ala Lys Thr Ala Glu
   625 630 635 640
   Met Thr Ser Pro Leu Asn Ala Ser Ala Val Ile Phe Val Asp Gln Thr
   645 650 655
   Lys Ala Ser Ile Thr Glu Ile Lys Ala Asp Lys Thr Thr Ala Lys Ala
   660 665 670
   Asp Gly Ser Asp Ala Ile Thr Tyr Thr Val Arg Val Met Lys Glu Gly
   675 680 685
   Ala Pro Val Val Asp Gln Lys Val Thr Phe Ser Lys Asp Phe Gly Thr
   690 695 700
   Leu Asn Lys Thr Glu Ala Thr Thr Asp Gln Asn Gly Tyr Ala Thr Val
   705 710 715 720
   Lys Leu Ser Ser Asn Thr Pro Gly Lys Ala Ile Val Ser Ala Lys Val
   725 730 735
   Ser Gly Val Gly Thr Glu Val Lys Ala Thr Thr Val Glu Phe Phe Ala
   740 745 750
   Pro Leu Ser Ile Asp Gly Asp Lys Val Thr Val Ile Gly Thr Gly Ile
           755                 760                 765
   Thr Gly Ala Leu Pro Lys Asn Trp Leu Gln Tyr Gly Gln Val Lys Leu
       770                 775                 780
   Gln Ala Thr Gly Gly Asn Gly Lys Tyr Thr Trp Lys Ser Ser Asn Thr
   785                 790                 795                 800
   Lys Ile Ala Ser Val Asp Asn Ser Gly Val Ile Thr Leu Asn Glu Lys
                   805                 810                 815
   Gly Ser Ala Thr Ile Thr Val Val Ser Gly Asp Asn Gln Ser Ala Thr
               820                 825                 830
   Tyr Thr Ile Asn Ala Pro Gly Ser Ile Val Ile Ala Val Asp Lys Asn
           835                 840                 845
   Thr Arg Val Thr Tyr Phe Asp Ala Glu Asn Lys Cys Lys Thr Asn Ser
       850                 855                 860
   Ala Asn Leu Ala Gln Pro Lys Glu Leu Leu Ala Asn Ile Tyr Ser Thr
   865                 870                 875                 880
   Trp Gly Ala Ala Asn Lys Tyr Pro Tyr Tyr Ser Gly Ser Lys Ser Leu
                   885                 890                 895
   Thr Ala Trp Ile Lys Gln Ser Ser Ser Glu Gln Ser Ser Gly Val Ser
               900                 905                 910
   Ser Thr Tyr Asp Leu Val Thr Lys Asn Gln Leu Ile Asn Val Gly Val
           915                 920                 925
   Asn Asn Lys Asn Ala Phe Ser Val Cys Val Lys  *
       930                 935                 940
(2) INFORMATIONS POUR LA SEQ ID NO: 3:
   (i) CARACTERISTIQUES DE LA SEQUENCE:
      (A) LONGUEUR: 193 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 3:
(2) INFORMATIONS POUR LA SEQ ID NO: 4:
   (i) CARACTERISTIQUES DE LA SEQUENCE:
      (A) LONGUEUR: 381 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 4:
   Met Leu Asn Val Asn Ser Asp Ile Gln Ser Met Arg Ser Gly Ala Ser
    1               5                  10                  15
   Ala Ala Thr Ala Thr Ser Gly Ile Asn Gln Pro Glu Val Thr Ser Ala
               20                  25                  30
   Leu Asp Leu Gln Leu Val Lys Ser Thr Ala Pro Ser Ala Ser Trp Thr
           35                  40                  45
   Glu Ser Thr Ala Leu Pro Thr Pro Pro Ala Gly His Ser Leu Val Thr
       50                  55                  60
   Pro Ser Val Ala Glu Asp Val Leu Ser Lys Leu Phe Gly Gly Ile Ser
   65                  70                  75                  80
   Gly Glu Val Arg Ser Arg Thr Glu Gly Thr Glu Pro Gln Arg Ser Thr
                   85                  90                  95
   Gln Asn Ala Ser Ser Gly Tyr Pro Tyr Leu Ser Gln Val Asn Asn Val
               100                 105                 110
   Asp Pro Gln Ala Met Met Met Met Val Thr Leu Leu Ser Leu Asp Ala
           115                 120                 125
   Ser Ala Gln Lys Val Ala Ser Met Lys Asn Ser Asn Glu Ile Tyr Thr
       130                 135                 140
   Asp Gly Gln Asn Lys Ala Leu Asp Asn Lys Thr Leu Glu Phe Lys Lys
   145                 150                 155                 160
   Gln Leu Glu Glu Gln Gln Lys Ala Glu Glu Lys Ala Gln Lys Ser Lys
                   165                 170                 175
   Ile Leu Gly Gln Val Phe Gly Trp Leu Gly Val Ala Val Thr Ala Ile
               180                 185                 190
   Ala Ala Ile Phe Asn Pro Ala Leu Trp Ala Val Val Ala Val Ser Ala
           195                 200                 205
   Thr Ala Met Ala Leu Gln Thr Ala Val Asp Val Met Gly Asp Lys Ala
       210                 215                 220
   Pro Gln Ala Leu Lys Thr Ala Ala Gln Val Phe Gly Gly Val Ser Ile
   225                 230                 235                 240
   Ala Ala Ser Ile Leu Thr Ala Gly Ile Gly Gly Val Ser Ser Leu Met
                   245                 250                 255
   Ser Lys Val Gly Asp Val Ala Asn Lys Val Gly Ser Asn Ile Val Lys
               260                 265                 270
   Thr Val Thr Lys Leu Ala Asp Val Phe Val Glu Asn Val Val Ser Lys
           275                 280                 285
   Val Ala Ala Thr Ala Asn Gly Phe Thr Thr Ser Ala Arg Ser Ile Gly
       290                 295                 300
   Thr Thr Val Leu Asn Asn Asp Ser Ala Gln Tyr Ser Ile Leu Ala Gln
   305                 310                 315                 320
   Leu Ser Ala Tyr Ala Val Gln Asn Leu Thr Arg Gln Ser Glu Asn Leu
                   325                 330                 335
   Gly Glu Ser Ala Lys Val Glu Leu Asp Lys Ala Ala Ala Glu Leu Arg
               340                 345                 350
   Asn Gln Ala Ser Tyr Leu Gln Asn Ala Ser Gln Leu Ile Ser Asp Ser
           355                 360                 365
   Ala Arg Val Asn Ser Arg Ile Val Ser Gly Arg Val  *
       370                 375                 380
(2) INFORMATIONS POUR LA SEQ ID NO: 5:
   (i) CARACTERISTIQUES DE LA SEQUENCE:
      (A) LONGUEUR: 315 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 5:
   Met Asn Thr Ile Asp Tyr Thr Asn Gln Val Met Thr Val Asn Ser Val
   1 5 10 15
   Ser Glu Asn Thr Thr Gly Ser Asn Ala Ile Thr Ala Ser Ala Ile Asn
   20 25 30
   Ser Ser Leu Leu Thr Asp Gly Lys Val Asp Val Ser Lys Leu Met Leu
   35 40 45
   Glu Ile Gln Lys Leu Leu Gly Lys Met Val Arg Ile Leu Gln Asp Tyr
   50 55 60
   Gln Gln Gln Gln Leu Ser Gln Ser Tyr Gln Ile Gln Leu Ala Val Phe
   65 70 75 80
   Glu Ser Gln Asn Lys Ala Ile Asp Glu Lys Lys Ala Ala Ala Thr Ala
   85 90 95
   Ala Leu Val Gly Gly Ala Ile Ser Ser Val Leu Gly Ile Leu Gly Ser
   100 105 110
   Phe Ala Ala Ile Asn Ser Ala Thr Lys Gly Ala Ser Asp Ile Ala Gln
   115 120 125
   Lys Thr Ala Ser Thr Ser Ser Lys Ala Ile Asp Ala Ala Ser Asp Thr
   130 135 140
   Ala Thr Lys Thr Leu Thr Lys Ala Thr Glu Ser Val Ala Asp Ala Val
   145 150 155 160
   Glu Asp Ala Ser Ser Val Met Gln Gln Ala Met Thr Thr Ala Thr Arg
   165 170 175
   Ala Ala Ser Arg Thr Ser Asp Val Ala Asp Asp Ile Ala Asp Ser Ala
   180 185 190
   Gln Arg Ala Ser Gln Leu Ala Glu Asn Ala Ala Asp Ala Ala Gln Lys
   195 200 205
   Ala Ser Arg Ala Ser Arg Phe Met Ala Ala Val Asp Lys Ile Thr Gly
   210 215 220
   Ser Thr Pro Phe Ile Ala Val Thr Ser Leu Ala Glu Gly Thr Lys Thr
   225 230 235 240
   Leu Pro Thr Thr Val Ser Glu Ser Val Lys Ser Asn His Glu Ile Ser
   245 250 255
   Glu Gln Arg Tyr Lys Ser Val Glu Asn Phe Gln Gln Gly Asn Leu Asp
   260 265 270
   Leu Tyr Lys Gln Glu Val Arg Arg Ala Gln Asp Asp Ile Ala Ser Arg
           275                 280                 285
   Leu Arg Asp Met Thr Thr Ala Ala Arg Asp Leu Thr Asp Leu Gln Asn
       290                 295                 300
   Arg Met Gly Gln Ser Val Arg Leu Ala Gly  *
   305                 310                 315

## Description

L'invention est relative à de nouveaux mutants non pathogènes d'*E. coli,* utilisables en particulier pour l'obtention de vaccins permettant la prévention de la colibacillose chez le lapereau.

La colibacillose est la principale cause de diarrhée chez les lapereaux au sevrage. Cette maladie est une cause importante de mortalité dans les élevages, et des recherches ont été entreprises en vue d'obtenir un vaccin efficace. Les principales stratégies vaccinales développées jusqu'à présent font appel à des souches homologues inactivées ou à des souches hétérologues vivantes non ou peu pathogènes induisant des réponses immunitaires locales (inductions d'immunoglobulines A anti-lipopolysaccharide) et/ou des effets de barrière écologique s'exerçant à l'égard des souches 0103 pathogènes.

Cependant, aucune de ces stratégies n'a abouti jusqu'à présent à l'obtention d'un vaccin conférant une protection totale et d'une parfaite innocuité. Des recherches sont donc poursuivies en vue d'identifier les mécanismes responsables de la pathogénicité de certaines souches d'*E. coli*.

Des souches d'*E. coli* entéropathogènes (EPEC) de l'homme, qui sont responsables de diarrhées chez l'enfant, sont capables de provoquer la formation de lésions spécifiques de l'épithélium intestinal dites : "lésions d'attachement-effacement".

Chez ces souches EPEC humaines, on a défini un phénotype d'attachement-effacement, corrélé à un test *in vitro* sur cellules épithéliales de lignée Hela ou Hep-2: le test FAS (pour "Fluorescent Actin Staining"). Dans ce test, les bactéries qui adhèrent aux cellules de manière dite localisée, provoquent au niveau des foyers d'adhésion une concrétion d'actine polymérisée qui est détectée par la phalloidine couplée à un fluorochrome [KNUTTON et al., Infect. Immun. 57, p. 1290-1298, (1989)]. Les déterminants majeurs de ce phénotype d'attachement-effacement sont codés par un locus dénommé LEE ("Locus of Enterocyte Effacement") [Mc Daniel et al., Proc. Natl. Acad. Sci. 92, p. 1664-1668, (1995)].

Il a été observé que des souches d'*E. coli* responsables des diarrhées des lapereaux peuvent provoquer des lésions d'attachement-effacement similaires à celles provoquées par les souches EPEC humaines [LICOIS et al., Infect. Immun. 59, P. 3796-3800, (1991); MOON et al., Infect. Immun., 41, p. 1340-1351, (1983); PEETERS et al. Vet. Pathol. 22, p. 54-59, (1985); TAKEUCHI et al., Infect. Immun., 19, p. 686-694, (1978)]. Ces souches EPEC de lapin sont également dénommées REPEC (pour "Rabbit EPEC") par certains auteurs [ROBINS-BROWNE et al., Infect. Immun. 62, p. 3329-3336, (1994); ROBINS-BROWNE et al., Infect. Immun. 62, p. 1584-1592, (1994)].

Un homologue du locus chromosomique LEE de 35 kpb originellement mis en évidence chez la souche EPEC prototype d'origine humaine E2348/69, a été retrouvé [KARAOLIS et al., In « Proceedings of the first International Rushmore Conference on Mechanisms in the Pathogenesis of Enteric Diseases ». Rapid City, S.D., (1995); Mc Daniel et al., Proc. Natl. Acad. Sci. 92, p. 1664-1668, (1995)] chez la souche REPEC de référence dénommée RDEC-1 (sérovar 015:H-), initialement décrite par CANTEY et BLAKE [J. Infect. Dis. 135, p. 454-462, (1977)]

Une protéine de membrane externe de 95-100 kDa appelée intimine, codée par un gène dénommé *eaeA* du locus LEE des souches EPEC humaines participe à la formation des lésions d'attachement-effacement [JERSE et al., Infect. Immun. 59, p. 4302-4309, (1991)]. Une étude concernant le rôle du gène *eaeA* dans le pouvoir pathogène a été effectuée par DONNENBERG et al. [J. Clin. Invest., 92, p. 1412-1417, (1993)]. La souche E2348/69 a été testée chez 11 volontaires qui ont reçu 2.10¹⁰ UFC par voie orale. Onze autres volontaires ont reçu la même dose d'un mutant *eaeA* isogénique. Les résultats démontrent le rôle de *eaeA* dans la virulence, et donc indirectement une corrélation entre lésions A/E, FAS positif et locus LEE (non connu à l'époque) avec la virulence, puisqu'il était alors considéré que *eaeA* était seul impliqué dans les lésions A/E et le phénotype FAS positif. Cependant, le pouvoir pathogène résiduel du mutant démontre l'intervention de gènes de virulence autres que *eaeA.*

En outre, le gène *eaeA* est également présent chez des souches d'*E. coli* non-entéropathogènes [CANTEY et moseley, Infect. Immun. 59, p. 3924-3929, (1991) ; [LEROY et al. J. Med. Microbiol., 40, p. 90-94, (1993)].

Un analogue du gène *eaeA* qui code pour l'intimine a été mis en évidence chez différentes souches d'*E. coli* de lapin [AGIN et WOLF, In Proceedings of the First International Rushmore Conference on Mechanisms in the Pathogenesis of Enteric Diseases, Rapid City, SD. p. 43 (1995); LEROY et al. J. Med. Microbiol. 40, p. 90-94, (1993); POHL et al., Infect. Immun., 61, p. 2203-2206, (1993)].

En Europe de l'Ouest, des études épidémiologiques ont montré que les souches responsables des diarrhées au sevrage du lapin appartiennent à plusieurs sérogroupes, le plus fréquent étant 0103 [BLANCO et al., Vet. Microbiol., 38, p. 193-201, (1994); CANGUILHEM et MILON, J. Clin. Microbiol. 27, p. 743-747, (1989)].

Les souches 0103, (qui possèdent en outre la particularité de ne pas fermenter le rhamnose), sont très pathogènes chez le lapin après inoculation expérimentale par voie digestive, et produisent des lésions d'attachement-effacement [LICOIS et al. Infect. Immun. 59, p. 3796-3800, (1991); POHL et al., Infect. Immun., 61, p. 2203-2206, (1993); ROBINS-BROWNE et al., Infect. Immun., 62, p. 3329-3336, (1994); ROBINS-BROWNE et al., Infect. Immun. 62, p. 1584-1592, (1994)] d'aspect similaire à celles observées avec les souches EPEC d'origine humaine. Ce critère lésionnel, associé au fait que l'ADN chromosomique de ces souches hybride avec des sondes spécifiques du gène *eaeA* de la souche EPEC humaine de référence [LEROY et al. J. Med. Microbiol., 40, p. 90-94, (1993); POHL et al., Infect. Immun., 61, P. 2203-2206, (1993)] comme dans le cas de la souche RDEC-1, permettrait de les rapprocher des EPEC (ou REPEC) . Toutefois, aucune homologie avec des gènes du locus LEE autres que le gène *eaeA* n'a été mise en évidence chez les souches 0103.

Les souches d'*E. coli* 0103 produisent une adhésine spécifique, dénommée AF/R2, qui permet aux bactéries d'adhérer aux entérocytes de lapin et aux cellules de lignée Hela et Hep-2 [MILON et al., Infect. Immun. 58, p. 2690-2696, (1990)].

Il a été démontré que cette adhésine contribuait au pouvoir pathogène pour le lapereau des souches 0103. Cette démonstration a été effectuée en comparant le pouvoir pathogène d'une souche représentative du sérogroupe 0103, dénommée souche B10, à celui d'un mutant de cette souche ayant perdu la propriété d'adhésion par suite de l'insertion du transposon *Tn5*::*phoA* dans l'opéron codant pour l'adhésine AF/R2 [PILLIEN et al., Vet. Microbiol., 50, p. 105-115, (1996)]. L'utilisation vaccinale de ces mutants a été proposée [CHALARENG et al., Communication aux VI^{èmes} journées de la recherche cunicole, La Rochelle, 6 et 7 décembre 1994 ; INRA-ITAVI-ASFC Publ.]

Afin de préciser le degré de similitude entre les souches *E. coli* 0103 et les souches EPEC d'origine humaine, les inventeurs ont étudié la réponse des souches 0103 au test FAS ; le phénomène de FAS est en effet considéré comme associé au locus LEE des EPEC [Mc DANIEL et al. Proc. Natl. Acad. Sci. 92, p. 1664-1668, (1995)].

Or, les Inventeurs ont constaté que la souche B10 répondait négativement à ce test FAS, alors que, dans les mêmes conditions expérimentales, la souche REPEC RDEC-1, ainsi que les souches EPEC humaines produisaient des réactions positives.

En revanche, les Inventeurs ont constaté que, de manière surprenante, la souche B10 produisait sur les cellules épithéliales de lignée HeLa un effet cytopathique (ECP) original, se manifestant par une réorganisation du cytosquelette et des plaques d'adhésion focale. Comme dans le cas de la réponse de type FAS, cet ECP est associé à une polymérisation intense de l'actine cellulaire. Toutefois, contrairement à ce qui est observé dans les réponses de type FAS, l'actine polymérisée ne se localise pas au niveau des contacts bactériens, mais s'organise sous la forme de câbles traversant la cellule de part en part. Ces modifications du cytosquelette s'amplifient notablement et irréversiblement avec le temps.

En outre, la formation des câbles d'actine s'accompagne de l'augmentation progressive de la quantité de vinculine, un phénomène qui n'est pas observé dans la réponse de type FAS [FINLAY et al., Infect. Immun. 60, p. 2541-2543, (1992)]. Chez les cellules contrôle, la vinculine se présente sous la forme d'amas punctiformes apparaissant sur le contour des cellules, tandis que chez les cellules exposées à B10, la vinculine est distribuée sur toute la surface cellulaire, passant d'une distribution punctiforme à une distribution quasi-fibrillaire, calquée sur celle des câbles d'actine. Cette modification du cytosquelette s'accompagne d'un arrêt de la multiplication cellulaire et d'une augmentation modérée de la taille de cellules. La cytostase est durable mais débouche sur la mort des cellules à l'issue d'une période de 5 à 6 jours.

En testant d'autres souches, les Inventeurs ont également constaté que cet ECP se manifestait chez toutes les souches 0103 rhamnose négative de lapin, ainsi que chez la souche RDEC-1. Ils l'ont en outre également observé chez deux isolats cliniques EPEC d'origine humaine. Par contre, la souche de référence d'origine humaine E2348/69, testée dans les mêmes conditions, n'a pas produit d'ECP détectable.

Les modifications du cytosquelette qui caractérisent cet ECP évoquent celles produites par les toxine CNF et CNF2 d'*E. coli* [OSWALD et al., Proc. Natl. Acad. Sci. USA, 91, p. 3814-3818, (1994)]. Toutefois, les Inventeurs n'ont mis en évidence chez B10 aucun gène homologue à ceux de CNF1 et CNF2 d'*E. coli.* En outre, alors que l'activité des CNF est détectable dans les lysats de culture des souches produisant, contrairement aux souches produisant CNF, aucune activité n'a pu être détectée dans les lysats de culture de B10. Il est toutefois possible que le mécanisme d'action conduisant à l'ECP induit par B10 soit similaire à celui des toxines CNF, qui est associé à l'activation de Rho, une petite protéine G qui participe à la régulation du cytosquelette et des foyers d'adhésion focale [FLINN et RIDLEY, J. Cell Sci. 109, p. 1133-1141, (1996)].

Parallèlement, les Inventeurs ont procédé à l'hybridation de l'ADN chromosomique de plusieurs souches 0103, dont la souche prototype B10, avec quatre sondes dérivées du bloc de virulence LEE de 35 kb de la souche, et ont également comparé les protéines sécrétées dans le milieu de culture par les souches 0103 et par la souche E2348/69, par l'analyse de leurs profils électrophorétiques respectifs, et par immunoprécipitations croisées. Ils ont ainsi mis en évidence chez les souches 0103 un locus homologue du bloc de virulence LEE de E2348/69, bien que présentant avec ce dernier quelques différences de structure (détectées par polymorphisme des sites de restriction de l'ADN et des différences mineures de migration électrophorétique des protéines sécrétées). Ce locus analogue au LEE des souches EPEC sera ci-après dénommé également « locus LEE ».

Les Inventeurs ont en outre constaté que cet ECP était corrélé au pouvoir pathogène *in vivo* des souches 0103.

Les Inventeurs ont construit, à partir d'*E. coli* 0103, des souches portant des mutations de ce locus LEE, et ont constaté qu'une souche dans laquelle un des gènes de ce locus LEE est inactivé n'induit plus l'ECP, et perd son pouvoir pathogène, bien qu'elle conserve l'adhésine AF/R2, et demeure capable de coloniser l'intestin. Il apparaît donc que le locus LEE des souches 0103 porte des déterminants génétiques responsables de l'ECP et de la pathogénicité.

Enfin, les Inventeurs ont également étudié les propriétés immunogènes de ces mutants du locus LEE, et ont constaté que ces souches mutantes conféraient une protection très efficace contre la souche parentale pathogène.

La présente invention a pour objet un procédé d'obtention d'une souche mutante non-pathogène d'*E. coli* à partir d'une souche d'*E. coli* pathogène capable d'induire sur des cellules épithéliales HeLa un effet cytopathique se manifestant par la formation de câbles d'actine polymérisée traversant de part en part lesdites cellules et par une augmentation de la quantité de vinculine, caractérisé en ce que l'on procède à la mutagenèse de ladite souche d'*E. coli* pathogène, et en ce que l'on sélectionne les mutants ayant perdu la capacité d'induire ledit effet cytopathique.

A titre d'exemple de souche d'*E. coli* pathogène capable d'induire l'effet cytopathique mentionné ci-dessus, et donc utilisable comme matériau de départ pour la mise en oeuvre du procédé selon l'Invention, on citera la souche B10, qui a été déposée auprès de la Collection Nationale de Cultures de Microorganismes (CNCM), 28, rue du Docteur Roux, 75724 PARIS CEDEX 15, le 15 Janvier 1997 sous le n° I-1807

La présente invention englobe également les souches mutantes non-pathogènes d'*E*. *coli* appartenant au sérogroupe O103, qui sont susceptibles d'être obtenues par ledit procédé à partir d'une souche d'*E*. *coli* pathogène du lapin, du sérogroupe 0103. Ces souches portent au moins une mutation résultant en l'inactivation d'au moins l'un des gènes du locus LEE.

Selon un mode de réalisation préféré d'une souche mutante conforme à l'invention, elle porte au moins une mutation résultant en l'inactivation d'au moins l'un des gènes sep du locus LEE.

Une souche mutante conforme à l'invention, dénommée B10/CA1 a été déposée auprès de la Collection Nationale de Cultures de Microorganismes (CNCM), 28, rue du Docteur Roux, 75724 PARIS CEDEX 15, le 15 Janvier 1997 sous le n° I-1808.

La présente invention a également pour objet des vaccins, et en particulier des vaccins administrables par voie orale, comprenant au moins une souche mutante d'*E. coli* conforme à l'invention.

Avantageusement, ladite souche mutante possède une adhésine AF/R2 fonctionnelle, et conserve de ce fait sa capacité à coloniser l'intestin.

Par exemple, des vaccins conformes à l'invention, comprenant la souche B10/CA1, peuvent être administrés par voie orale, et permettent de protéger des lapereaux au sevrage contre les infections par des *E. coli* pathogènes du sérogroupe 103.

La présente invention sera mieux comprise à l'aide du complément de description qui va suivre, qui se réfère à des exemples non-limitatifs d'obtention de souches mutantes conformes à l'invention, et d'utilisation de ces souches en tant que vaccins.

### EXEMPLE 1 : MISE EN EVIDENCE DE L'EFFET CYTOPATHIQUE PRODUIT PAR LES SOUCHES D'E. COLI 0103 SUR LES CELLULES HELA EN CULTURE.

Les souches bactériennes utilisées dans cette expérimentation sont :
- les souches de référence d'*E. coli* entéropathogènes de lapin B10 et RDEC-1 ;
- la souche B10/16E1, qui est un mutant de B10 obtenu par mutagenèse *TnphoA,* et ne produisant plus l'adhésine AF/R2 [PILLIEN et al., Vet. Microbiol. 50, p. 105-115, (1996)].
- 18 souches *d'E. coli* isolées de cas séparés de diarrhées du lapin et étudiées antérieurement pour leur pouvoir pathogène expérimental et la possession de certains caractères de virulence, tels que AF/R2 et *eae*A [CANGUILHEM et MILON, J. Clin. Microbiol., 27, p. 743-747, (1989); LEROY et al., J. Med. Microbiol., 40, p. 90-94, (1993)];
et à titre de témoin, des souches humaines FAS positives :
- la souche de référence EPEC humaine E2348/69 ;
- deux souches humaines EPEC dénommées CF11201 et CF517 (aimablement fournies par Christiane FORESTIER, Faculté de Pharmacie de Clermont-Ferrand), provenant de diarrhées d'enfant, hybridant avec les sondes spécifiques de *eaeA* et *bfp* et répondant positivement au FAS test.

### - Test d'interaction sur les cellules HeLa

La veille du test d'interaction, les cellules HeLa sont ensemencées en lames à compartiments (LAB-TEK, MILES SCIENTIFIC) ou en plaques de titrage pour cultures cellulaires (NUNC), à raison de 4.10⁴ cellules par ml de milieu minimum essentiel de EAGLE avec sels de EARLE (MEME, GIBCO) contenant 10% de sérum de veau foetal (SVF, GIBCO) et 50 µg/ml de gentamicine (GIBCO). La veille du test également, les bactéries sont pré-cultivées en bouillon PENASSAY (DIFCO). Après 24 H d'incubation à 37°C en présence de 5% de C02, les cellules sont lavées au tampon salin de EARLE (EBSS, GIBCO) puis mises en présence des bactéries dans du MEME contenant 25 mM de tampon HEPES (GIBCO), 1% de D-mannose (SIGMA) et 5% de SVF (sérum de veau foetal). Après 4 h d'incubation à 37°C en présence de 5% de CO₂, les cellules sont lavées abondamment à l'EBSS puis remises en MEME contenant 10% de SVF, et 80 µg/ml de gentamicine pour détruire les bactéries résiduelles. Après des temps d'incubation variables (de 1 à 6 jours à 37°C) en présence de 5% de CO₂, les cellules sont lavées à l'EBSS, fixées au formol à 1% en PBS pendant 30 min. à température ambiante.

Les modifications morphologiques ont été recherchées, et la croissance cellulaire quantifiée à l'issue de ces différents temps d'incubation.

### * Modifications du cytosquelette :

L'actine-F est visualisée par la phalloïdine couplée à la rhodamine (MOLECULAR PROBES) dans les conditions recommandées par le fabricant. La vinculine est mise en évidence par immunofluorescence indirecte à l'aide d'un anticorps monoclonal de souris anti-vinculine (clone VIN-11-5; Sigma) à la dilution 1/100, et d'un anticorps de chèvre anti-souris couplé à la fluorescéine (n°0819, IMMUNOTECH). Les lames sont observées avec un microscope à fluorescence LEICA DMR (objectif x40).

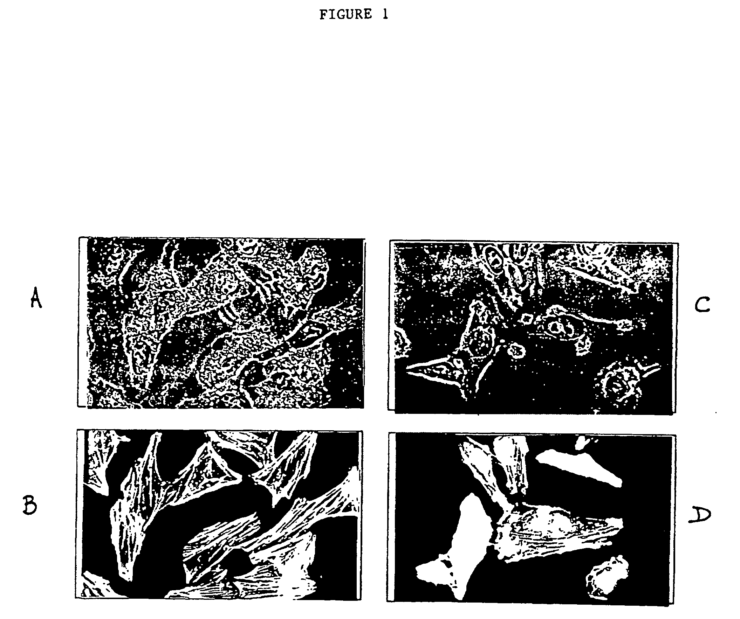

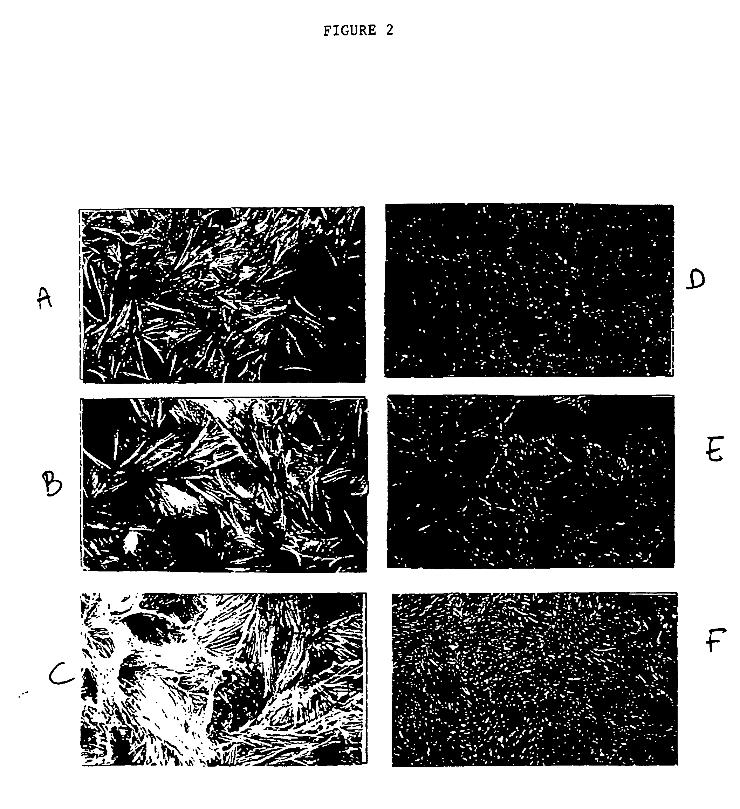

Les résultats sont illustrés par les Figures 1 et 2.

La Figure 1 représente les modifications morphologiques du cytosquelette des cellules HeLa en FAS test après exposition aux souches d'*E. coli* B10(A et B) et E2348/69 (C et D), suivie de 4 heures d'incubation.

Coloration de l'actine-F par la phalloïdine-rhodamine. Pour chaque souche un même champ microscopique est montré en contraste de phase (A et C) et en fluorescence (B et D). Objectif x40. Barre = 20 µm.

La Figure 2 représente les modifications du cytosquelette et des plaques d'adhésion focale induites dans les cellules HeLa à la suite de l'exposition à la souche d'*E. coli* B10, 24h (B et E), et 48h (C et F) après l'interaction, ou au mutant B10/CA1 (obtenu comme décrit à l'Exemple 2 ci-après), 24h après l'interaction (A et D).

A gauche (A,B,C) : coloration de l'actine-F par la phalloïdine-rhodamine.

A droite (D,E,F) : coloration de la vinculine par immunofluorescence indirecte avec des anticorps anti-vinculine.
Barre = 20 µm.

### * Quantification de la croissance cellulaire :

Le bleu de méthylène colore spécifiquement les protéines cellulaires et permet d'une part, d'observer la morphologie des cellules et, d'autre part, de doser le contenu protéique total des tapis cellulaires. Les protéines cellulaires sont colorées par le bleu de méthylène à 1% en tampon borate 0,01 M selon la méthode décrite par WILSON [Animal cell culture : A practical approach. R.I. Frishney (ed.) IRL Press Oxford, (1992)]. La quantification des protéines résiduelles des tapis cellulaires est effectuée le jour de l'interaction et 72 heures plus tard, par extraction du colorant par HCl 0,1 M, puis mesure de la densité optique à 630 nm sur un lecteur automatique de plaque ELISA (Modèle M 5000/7000, DYNATECH).

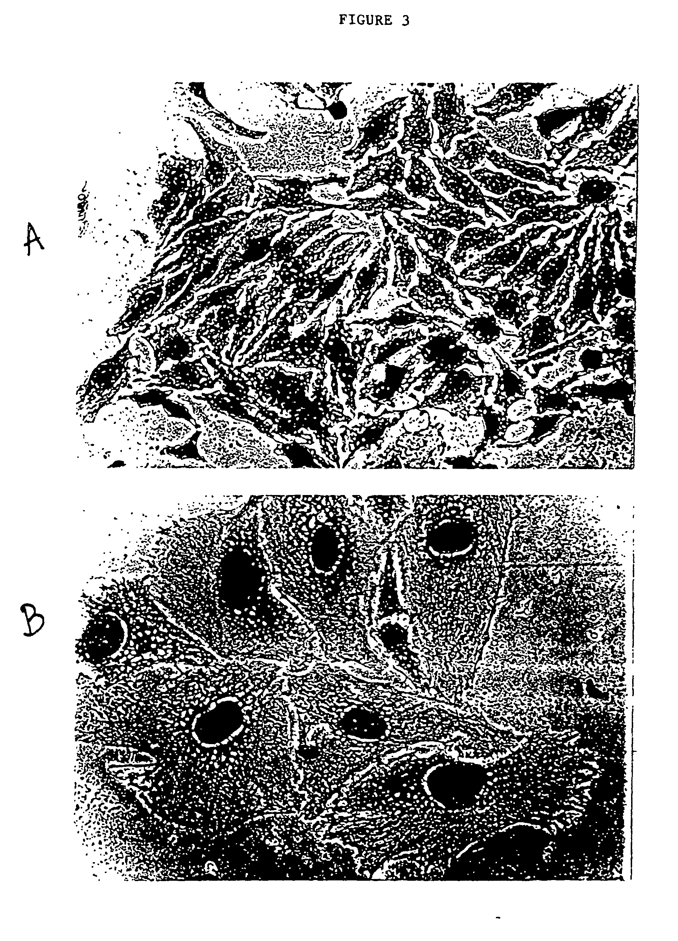

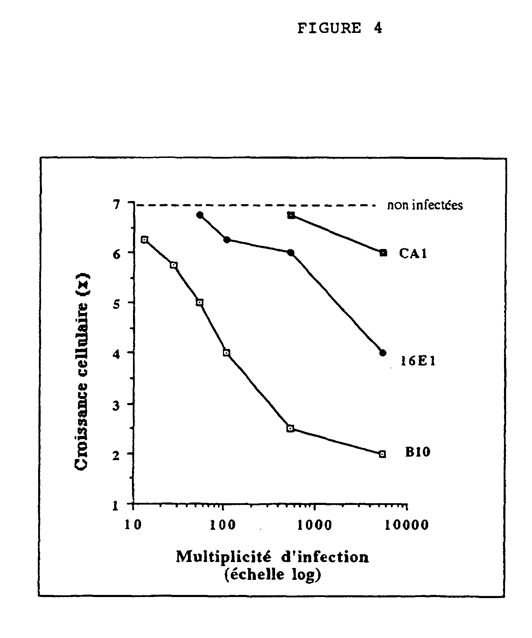

Les résultats sont illustrés par les Figures 3 et 4.

La Figure 3 illustre la morphologie des cellules HeLa quatre jours après l'interaction avec la souche B10 (B), ainsi qu'avec le mutant B10/CA1 (A). Coloration au bleu de méthylène. Barre = 20 µm.

La Figure 4 illustre l'effet de la dose d'inoculum bactérien sur la croissance des cellules Hela, à la suite de l'interaction avec les souches d'*E. coli* B10 (□), B10/CA1 (■), et B10/16E1 (●).

La multiplicité d'infection = nombre d'UFC par cellule au début du test d'interaction, est portée en abscisse ;

Le coefficient de croissance cellulaire = DO 72H après l'interaction/DO le jour de l'interaction est porté en ordonnée.

Chaque point représente la moyenne de quatre puits. L'écart-type du coefficient de croissance cellulaire est dans tous les cas inférieur à 0,2.

### Conclusion

Comme le montre la figure 1, la souche B10 n'induit pas de réponse de type FAS, c'est à dire ne provoque aucune concrétion significative d'actine polymérisée au point de contact des bactéries avec les cellules, et ceci en dépit d'une forte adhésion, contrairement à la souche E2348/69 qui exerce dans les mêmes conditions une réponse FAS fortement positive.

Une réponse de type FAS positive a également été observée avec la souche RDEC-1, malgré une adhésion très clairsemée, ainsi qu'avec les souches humaines CF11201 et CF517.

Parmi les autres souches d'*E. coli* de lapin testées, 4 souches de lapin de phénotype 0103 rhamnose négatives n'induisent pas de réponse de type FAS.

Les cellules exposées à la souche B10 présentent cependant de profondes modifications du cytosquelette décelables après la fin de l'interaction (figure 1), et s'amplifiant avec le temps (figures 2B, 2C, 2E et 2F). Ces modifications, désignées ici sous le terme d'ECP, se traduisent par le développement de nombreux câbles d'actine polymérisée traversant la cellule sous la forme de faisceaux souvent parallèles (figures 2B et 2C). Parallèlement au renforcement des faisceaux d'actine, on observe une augmentation progressive des formations de vinculine associées aux plaques d'adhésion focale. Ces formations qui sont habituellement punctiformes et périphériques chez des cellules exposées à la souche E2348/69, adoptent une distribution généralisée et pseudo-fibrillaire chez les cellules exposées à la souche B10, après 48 à 72H d'incubation (figure 2 E et 2 F).

La taille des cellules exposées à la souche B10 augmente sensiblement avec le temps, d'un facteur d'environ 2,5 après 4 jours d'incubation et selon le diamètre du noyau (figure 3) ; parallèlement, la croissance cellulaire est bloquée, comme l'indique le dosage de protéines totales dans les tapis résiduels (figure 4). La mortalité des cellules n'intervient qu'à partir du 5ème jour d'incubation.

On a d'autre part estimé qu'un inoculum de 50 à 100 unités formant colonies (UFC) de B10 par cellule en début de test permettait d'obtenir un ECP sur au moins 50% de cellules.

La souche mutante B10/16E1 ne produisant pas l'adhésine fimbriaire AF/R2 [PILLIEN et al., Vet. Microbiol., 50, p. 105-115, (1996)], produit le même ECP que la souche B10 parentale, mais, comme le montre la figure 4, son pouvoir cytopathique est environ 50 fois moins élevé.

Parmi les autres souches provenant de diarrhée de lapin qui ont été testées, les onze souches présentant le même phénotype que B10 (sérogroupe 0103, absence de fermentation du rhamnose, *eaeA* positive) produisent toutes les modifications morphologiques définissant l'ECP. Celui-ci est également induit par trois des six autres souches testées, ainsi que par la souche de référence RDEC-1. Parmi les souches EPEC d'origine humaine, la souche E2348/69 n'a pas produit d'ECP détectable tandis que les deux souches CF11201 et CF517 étaient fortement positives pour cet ECP.

Il n'a pas été possible de reproduire l'ECP avec des surnageants de culture d'interaction de B10 concentrés dix fois, ni avec des lysats de corps bactériens produits à partir des mêmes cultures et concentrés également dix fois.

### EXEMPLE 2 :PRODUCTION, SELECTION ET ANALYSE GENETIQUE DE MUTANTS ECP-NEGATIFS

Pour aborder le déterminisme moléculaire de cet ECP et examiner la corrélation éventuelle de l'ECP avec le pouvoir entéropathogène chez le lapin, des clones ayant perdu la capacité de produire l'ECP ont été isolés à partir d'une banque de mutants de B10 obtenus par insertion au hasard du transposon Tn5IS50_{L}::*phoA* (T*nphoA*) [MANOIL et BECKWITH, Proc. Natl. Acad. Sci. 82, p. 8129-8133, (1985)].

Tn*pho*A est introduit dans la souche B10 par conjugaison du plasmide suicide pRT733 hébergé par la souche *E. coli* SM10 (λpir+) [TAYLOR et al., J. Bacteriol. 171, p. 1870-1878, (1989)].

Les clones ayant reçu le transposon sont sélectionnés en deux temps :
1) Les mélanges de conjugaison sur gélose Luria-Bertani (milieu LA) sont d'abord enrichis en clones transposés par ensemencement en bouillon minimal M9 contenant de la kanamycine (20 µg/ml), puis incubation à 37°C pendant 48h. Dans ce milieu, les souches parentales ne poussent pas, SM10 étant auxotrophe *(thy thr leu)* et B10 sensible à la kanamycine.
2) Dans un deuxième temps, les cultures d'enrichissement sont étalées sur du milieu LA contenant de la kanamycine (50 µg/ml) et du 5-bromo-4-chloro-3-indolyl-phosphate (XP) (40 µg/ml). Les clones de couleur bleue sont alors répliqués sur de la gélose au rouge de phénol contenant 1% de L-rhamnose, et sur du milieu LA contenant de la kanamycine (100 µg/ml) ou de l'ampicilline (100 µg/ml) de manière à éliminer les clones dans lesquels le plasmide pRT733 dans son entier a été co-intégré au génome de B10.

Les clones possédant le phénotype: rhamnose-négatif, résistance à la kanamycine et sensibilité à l'ampicilline sont conservés pour l'analyse ultérieure.

Les mutants ainsi sélectionnés sont alors cultivés en milieu PENASSAY contenant de la kanamycine (50µg/ml) pendant une nuit puis conservés à -20°C après addition de glycérol (20% final). La détection de leur aptitude à produire l'ECP sur les cellules HeLa est réalisée ensuite sur plaques de cultures cellulaires à 96 puits (FALCON) selon les modalités décrites à l'exemple 1 ci-dessus.

Sur environ 2700 clones testés, 7 clones indépendants (c'est à dire issus de mélanges de conjugaison séparés) étaient ECP-négatifs et n'avaient pas intégré le plasmide suicide dans son intégralité.

La perte de l'ECP par l'un de ces mutants (B10/CA1) est illustrée par la Figure 2 (A et B), qui montre que les cellules exposées à ce mutant ne subissent pas les modifications du cytosquelette caractéristiques de l'ECP induit par la souche sauvage B10.

La figure 3A montre également que le mutant B10/CA1 ne provoque pas les modifications morphologiques induites par la souche B10. La figure 4 montre que le pouvoir cytopathique de ce mutant est quasiment nul.

### Caractérisation génétique des mutants ECP-négatifs.

Les techniques utilisées sont, sauf indication contraire, mises en oeuvre selon les protocoles standard décrits par [SAMBROOK et al., Molecular Cloning : A Laboratory Manual. Cold Spring Harbor, N.Y., (1989)].

Pour déterminer le nombre d'insertions dans les mutants ECP-négatifs, un fragment HindIII de 3,4 kpb de Tn5 a été couplé à la digoxigénine (DIG-UTP) par un kit commercial (DIG DNA, BOEHRINGER), en suivant les recommandations du fabricant. Cette sonde a été testée en transfert de Southern contre les fragments de digestion ClaI, BamHI et SalI de l'ADN total.

Deux des 7 clones sélectionnés précédemment ont été éliminés car ils contenaient une double insertion.

La localisation des inserts des 5 mutants restant a été effectuée comme suit :

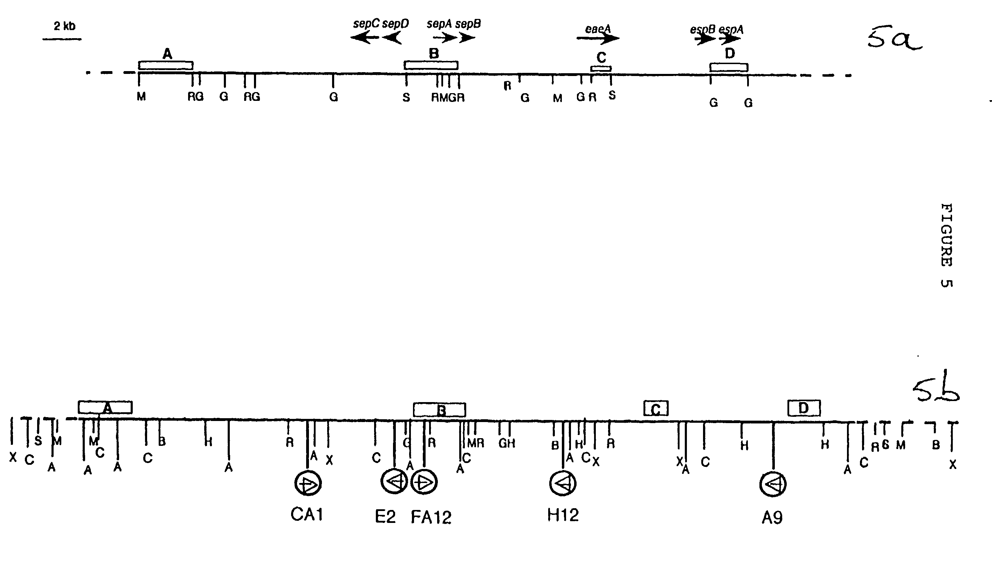

Les Inventeurs ont observé que l'ADN chromosomique de B10 s'hybridait avec quatre fragments spécifiques (A, B, C, D représentés sur la Figure 5) du locus LEE de la souche E2348/69 décrit par Mc DANIEL et al., [Proc. Natl. Acad. Sci. 92, p. 1664-1668, (1995)], et représenté sur la figure 5a. Cette hybridation s'effectue au niveau d'une région de plus de 35 kb (représentée sur la Figure 5b) qui apparaît donc comme constituant un locus analogue au LEE de E2348/69, avec toutefois des différences importantes dans le polymorphisme de restriction.

Des sondes ont été fabriquées en marquant les fragments A, B, C, D à la digoxine-UTP. Après digestion de l'ADN total par diverses enzymes de restriction puis Southern Blot, les profils d'hybridation des mutants ECP-négatifs de B10 avec ces sondes sont comparés à celui de la souche parentale B10 et à celui de E2348/69.

L'utilisation de ces sondes a permis de conclure que les cinq mutants ont reçu l'insert TnphoA dans la région correspondant au locus LEE. L'emplacement de l'insertion TnphoA pour ces 5 mutants est indiqué sur la Figure 5b par une flèche entourée d'un cercle.

Pour 3 d'entre eux (B10/CA1, B10/E2, B10/FA12), l'insert a été localisé dans un fragment EcoRI de 8 kb hybridant avec la sonde B, c'est à dire au voisinage de gènes homologues aux gènes de sécrétion *(sepA* à *sepD).* L'insert du mutant B10/H12 se situe entre les régions hybridant avec les sondes B et C, et celui du mutant B10/A9 dans le voisinage de gènes analogues à *espA* et *espB*.

### EXEMPLE 3 : COMPARAISON DES PROFILS DE PROTEINES DES SOUCHES INDUISANT UNE REPONSE FAS, DES SOUCHES INDUISANT UN ECP, ET DES MUTANTS ECP-NEGATIFS.

Afin d'identifier d'éventuelles protéines bactériennes sécrétées associées à l'ECP, les profils de migration électrophorétique des protéines de surnageant de culture ont été analysés chez la souche EPEC humaine E2348/69, chez les souches B10, RDEC-1 et B10/16E1, et chez les mutants ECP-négatif de B10.

Les bactéries sont cultivées dans les conditions du test d'interaction décrit à l'exemple 1 ci-dessus, mais en l'absence de cellules, et avec un inoculum de départ d'environ 10⁷ UFC par ml. Après 2h d'incubation, les bactéries sont centrifugées (4000 g, 15 min., 20°C) et suspendues dans 1 ml de MEME sans méthionine (n° 31900-020, GIBCO) additionné de 5% de SVF dialysé. Après 20 min. d'incubation à 37°C, pour épuiser le stock de méthionine endogène, de la méthionine marquée ³⁵S est additionnée à raison de 100 µCi/ml. Après 1h d'incubation supplémentaire, les bactéries sont éliminées par deux centrifugations successives (12 000 g, 10 min.) et les protéines du surnageant de centrifugation sont alors précipitées par le polyéthylène glycol (PEG) 4000 (200 mg/ml) (MERCK). Après 1h d'incubation à 0°C, une nouvelle centrifugation est effectuée (12 000 g, 30 min). Le culot de centrifugation est suspendu dans 30 µl de tampon de charge d'électrophorèse et les protéines sont séparées par migration électrophorétique en gel de polyacrylamide à 12% en présence de SDS (SDS-PAGE). Le gel est séché sous vide puis mis en contact pendant 6 jours avec un film β-Max (AMERSHAM), à température ambiante. La révélation se fait selon la méthode standard.

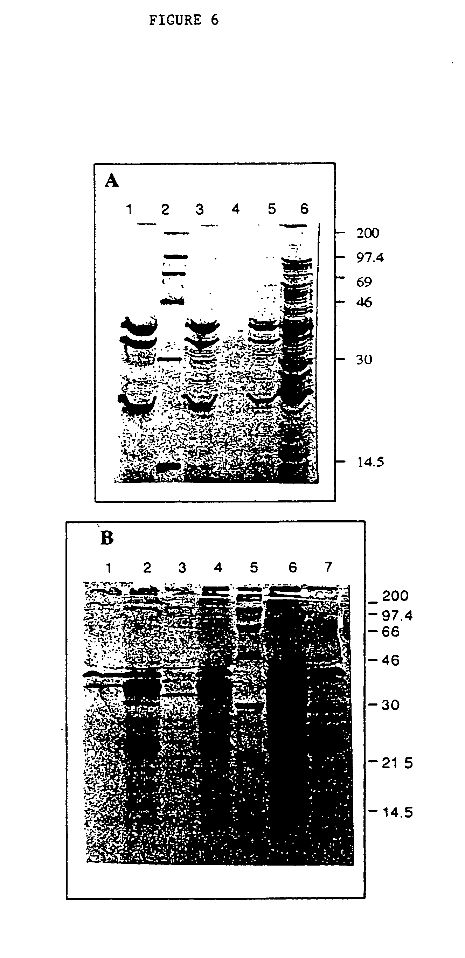

Les résultats sont illustrés par la Figure 6A.

Chez la souche parentale B10 (piste 3), trois protéines majeures, de poids moléculaires respectifs d'environ 39, 37 et 25 kDa, ainsi qu'une bande moins importante à 40 kDa sont identifiées. Le profil de la souche de lapin RDEC-1 (piste 1), et celui du mutant B10/16El sont identiques à celui de B10, tandis que celui de la souche EPEC de référence humaine E2348/69 (piste 6) présente quatre bandes de poids moléculaires assez proches (40, 39, 36 et 26 kDa), ainsi qu'une bande majeure supplémentaire d'environ 29 kDa qui n'a pas d'équivalent dans le profil de B10 (Figure 6A). Aucune de ces bandes n'apparaît chez le mutant ECP-négatif B10/CA1 (piste 4). La piste 2 contient les marqueurs de poids moléculaire.

Ces protéines ont également été analysées sur la base de leur parenté immunologique, en utilisant un sérum polyclonal de lapin produit contre les protéines de surnageant de B10 précipitées par le PEG.

L'immunoprécipitation avec l'antisérum contre les protéines de surnageant est réalisée sur les surnageants de culture après marquage au ³⁵S, comme décrit plus haut. L'immunoprécipitation est mise en oeuvre selon les modalités standard [SAMBROOK et al., Molecular Cloning : A Laboratory Manual. Cold Spring Harbor, N.Y., (1989)].

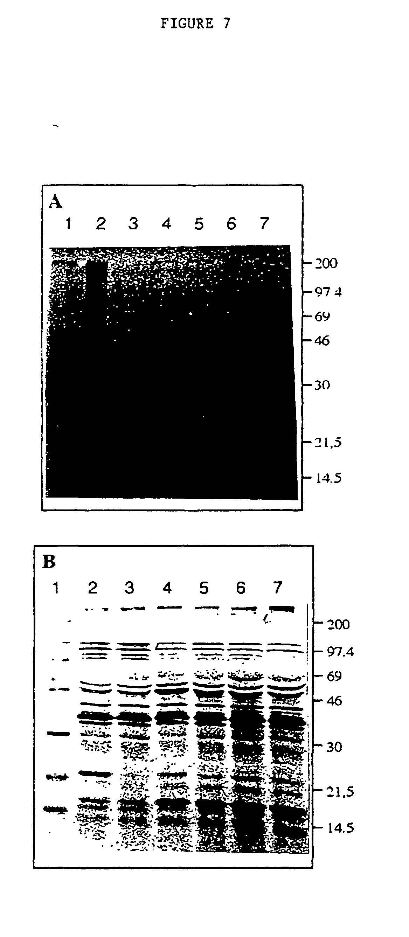

Les résultats sont illustrés par les figures 6 B et 7.

Légende de la Figure 6B : Pistes 1: B10 avec le sérum pré-immun; 2: B10; 3: B10/CA1, 4: B10/16E1; 5: marqueurs de poids moléculaires; 6: RDEC-1; 7: E2348/69 .

La figure 6 B montre que l'antisérum permet de précipiter les trois protéines majeures(39, 37 et 25 kDa) de B10 (piste 3) et RDEC-1 (piste 6) ainsi que les quatre protéines majeures de E2348/69 (39, 36, 29 et 26 kDa). Les trois protéines majeures de B10 sont présentes dans le surnageant du mutant AF/R2 négatif B10/16E1 (piste 4), mais n'apparaissent pas dans celui du mutant ECP-négatif B10/CA1 (piste 3). Il convient de noter que le sérum pré-immun possède également un léger pouvoir immunoprécipitant pour la protéine mineure de 40 kDa de B10. Ces résultats indiquent que les protéines de surnageant de B10 sont homologues à celles de RDEC-1 et de E2348/69 (même si les poids moléculaires de ces dernières sont sensiblement différents), et que la production de ces protéines dans le surnageant est affectée chez un mutant ECP-négatif.

Afin de déterminer si cette perte des protéines de surnageant résultait d'un défaut d'expression des gènes correspondants, ou d'un défaut de sécrétion par la bactérie, les profils d'immunoprécipitation des protéines de surnageant de la souche B10 et de ses mutants ECP-négatifs CA1, FA12, E2, A9, et H12 ont été comparés avec les profils d'immunoprécipitation des lysats bactériens des mêmes cultures.

Les lysats sont obtenus par trois cycles de congélation-décongélation (-20°C à 20°C), suivis d'une centrifugation (12 000 g, 10 min.), puis de la récupération du surnageant de lyse. L'immunoprécipitation est effectuée, après marquage au ³⁵S, dans les mêmes conditions que celles utilisées pour les protéines de surnageant. L'antisérum est épuisé au préalable avec un lysat bactérien de la souche d'*E. coli* de laboratoire HB101, pour éliminer les réactions non spécifiques.

Les résultats sont illustrés par les figures 7A (profils d'immunoprécipitation des protéines de surnageant) et 7B (profils d'immunoprécipitation des lysats bactériens).

Légende de la Figure 7 A : Pistes 1 : B10 ; 2 : marqueurs de poids moléculaire ; 3: CA1 ; 4 : FA12 ; 5 : E2 ; 6 : A9; 7: H12.

La figure 7A montre que chez tous les mutants, on observe une diminution significative des protéines de surnageant par rapport à B10. Chez trois d'entre eux, à savoir CA1, E2, et A9, cette diminution apparaît plus accentuée. L'immunoprécipitation permet également de mettre en évidence une bande à environ 110 kDa, d'intensité sensiblement équivalente chez toutes les souches et qui n'est donc pas affectée par les mutations.

Légende de la Figure 7 B : Pistes 1: marqueurs de poids moléculaire; 2: B10; 3: CA1; 4: FAI2; 5: 2E; 6:A9; 7: H12.

La figure 7B montre que les protéines majeures décrites plus haut sont présentes en quantité sensiblement équivalentes dans les lysats de B10 et ceux des mutants, à l'exception du mutant CA1 qui apparaît plus particulièrement déficient pour la protéine de 25 kDa.

### EXEMPLE 4 : PERTE DU POUVOIR PATHOGENE CHEZ UN MUTANT ECP-NEGATIF DE B10

Afin d'examiner si la perte de l'ECP avait un impact sur la virulence, le pouvoir pathogène par voie orale chez le lapin de la souche B10 a été comparé à celui de l'un de ces mutants, en l'occurrence la souche B10/CA1.

Le pouvoir pathogène est testé sur des lapereaux de race New Zealand White (souche INRA 1077) âgés de 35 jours, sevrés à l'âge de 28 jours selon les modalités décrites par PILLIEN et al. [Vet. Microbiol. 50, p. 105-115, (1996)]. Cinquante et un lapins sont allotés en trois groupes homogènes sur la base du poids et de la portée d'origine, puis inoculés par voie orale avec 2.10⁷ UFC des souches d'épreuve, à savoir, respectivement: la souche parentale B10 (18 animaux), le mutant ECP-négatif B10/CA1 (18 animaux) et une souche de laboratoire K12 non pathogène BM21 (15 animaux) . L'enregistrement des épisodes de diarrhée, de la mortalité et du poids, ainsi que le dénombrement et typage des souches d'*E. coli* des fèces, sont effectués comme décrit par PILLIEN et al., (1996, publication précitée).

L'inoculation orale de la souche parentale B10 a provoqué une chute de poids chez les 18 animaux inoculés, la diarrhée chez 17, puis la mort chez 16 d'entre eux. Les décès se sont échelonnés du 3^{ème} au 12^{ème} jour suivant l'inoculation. Par comparaison, les animaux inoculés avec la même dose du mutant B10/CA1, comme ceux inoculés avec la souche d'*E. coli* K12, n'ont montré aucune chute de poids ni aucun signe clinique. Les courbes de poids enregistrés dans ces deux derniers groupes étaient quasiment identiques.

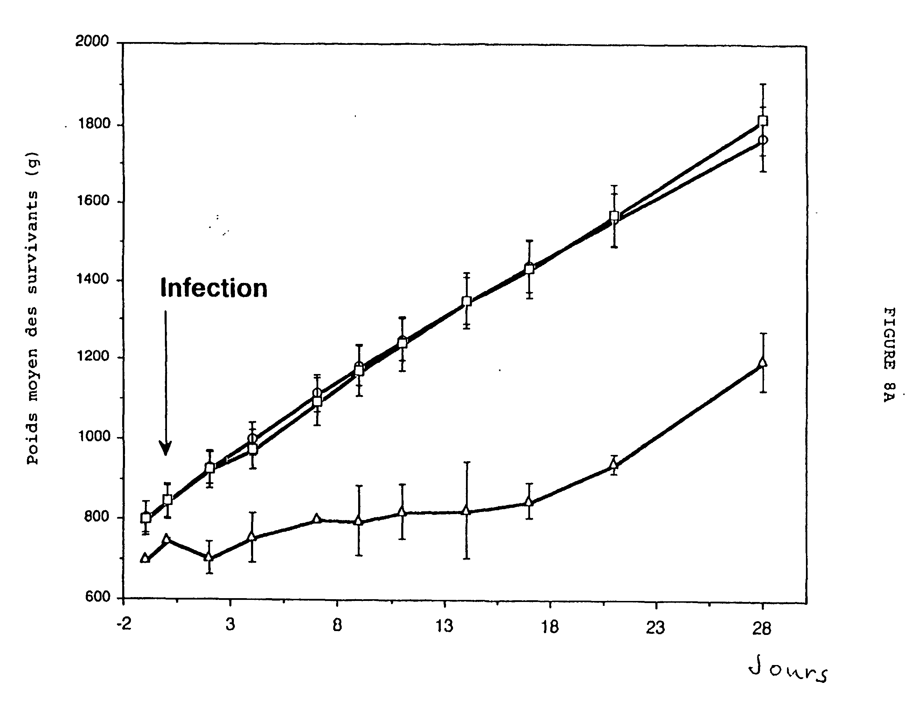

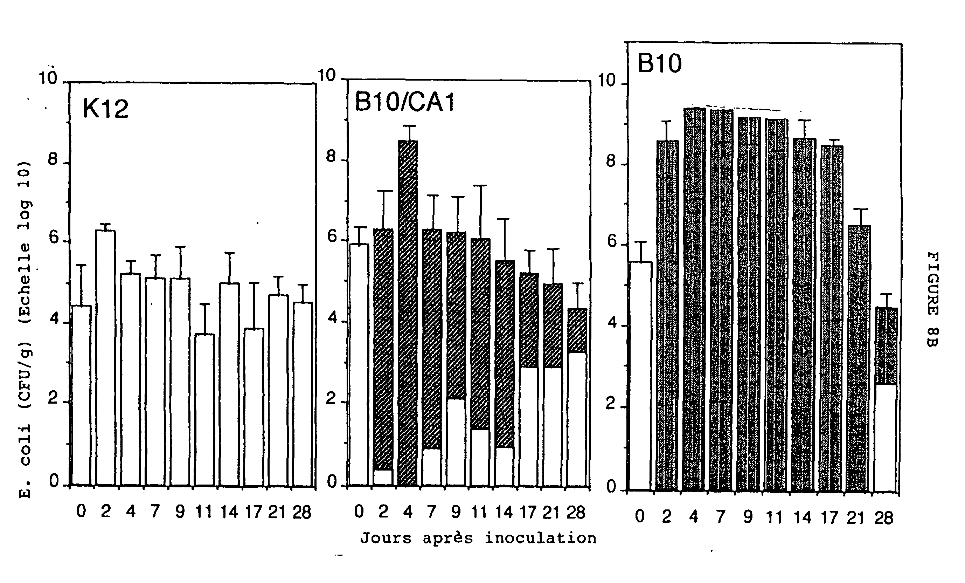

La figure 8A représente le poids moyen des animaux survivants dans chacun des groupes au cours de l'expérimentation : B10 (2 survivants/18 animaux) = Δ ; K12 (contrôle ; 15 survivants/15 animaux) = ○ ; B10/CA1 (18 survivants/18 animaux) = □ ;

La figure 8B représente la population colibacillaire fécale, à différents temps après l'inoculation, chez les animaux inoculés avec la souche contrôle K12, avec la souche B10 et avec la souche B10/CA1 :
□ : Flore colibacillaire identifiée comme constituée par la souche B10/CA1
□ : Flore colibacillaire identifiée comme constituée par la souche B10.

Chez les lapins inoculés avec la souche parentale B10, une colonisation colibacillaire massive a été observée, atteignant chez les survivants un niveau de 10⁹ UFC par g de contenu fécal, et ce pendant plus de deux semaines après l'inoculation. La totalité de la flore colibacillaire identifiée était constituée par la souche B10.

Ce niveau de population colibacillaire totale était 10³ à 10⁴ fois plus élevé que celui des lapins du groupe contrôle inoculés avec la souche d'*E. coli* K12, laquelle n'a d'ailleurs pas été détectée chez les animaux correspondants.

Chez les lapins inoculés avec le mutant B10/CA1, la souche a colonisé l'intestin à un niveau élevé puis la population colibacillaire s'est stabilisée à un niveau d'environ 10⁶ UFC par g de contenu fécal pendant au moins deux semaines. Pendant cette période, la souche B10/CA1 constituait de 70 à 100% de la population colibacillaire totale.

### EXEMPLE 5 : PROTECTION CONFEREE PAR UN MUTANT ECP-NEGATIF VIS A VIS D'UNE INFECTION PAR LA SOUCHE PARENTALE B10.

L'effet de l'inoculation orale d'une dose de 10⁴ CFU de la souche pathogène B10 a été comparé dans deux groupes de 18 lapereaux de 35 jours dont l'un avait reçu une semaine plus tôt la souche mutante B10/CA1 par voie orale (2.10⁷ CFU).

Trente six lapereaux (même race et mêmes conditions d'élevage que celles décrites à l'Exemple 4 ci-dessus) sont répartis en deux groupes homogènes sur la base du poids et de la portée d'origine. A l'âge de 30 jours, les animaux de l'un des deux groupes reçoivent par voie orale 2.10⁷ UFC du mutant B10/CA1 dans 2 ml de PBS. Sept jours plus tard, les animaux des deux groupes reçoivent la souche parentale pathogène B10 par voie orale, à la dose de 2.10⁴ UFC (c'est à dire environ 1 dose létale 50 %). L'enregistrement des épisodes de diarrhée, de la mortalité, et du poids, ainsi que le dénombrement et typage des souches d'*E. coli* des fèces sont effectués comme décrit à l'Exemple 4 ci-dessus.

Chez les lapereaux ayant reçu la souche mutante B10/CA1 par voie orale, aucune manifestation clinique, et aucune perte de poids n'ont été observées pendant toute la période d'observation.

En revanche, 11 des 18 lapins non vaccinés avec B10/CA1 ont présenté une chute de poids, 9 de la diarrhée, et 7 sur 18 sont morts.

L'analyse de la colonisation intestinale montre que la souche B10/CA1 a colonisé efficacement l'intestin jusqu'à l'administration de la souche parentale pathogène B10, puis s'est maintenue ensuite pendant deux semaines à un niveau stable d'environ 10⁷ CFU/g, empêchant l'installation de B10 à un niveau détectable.

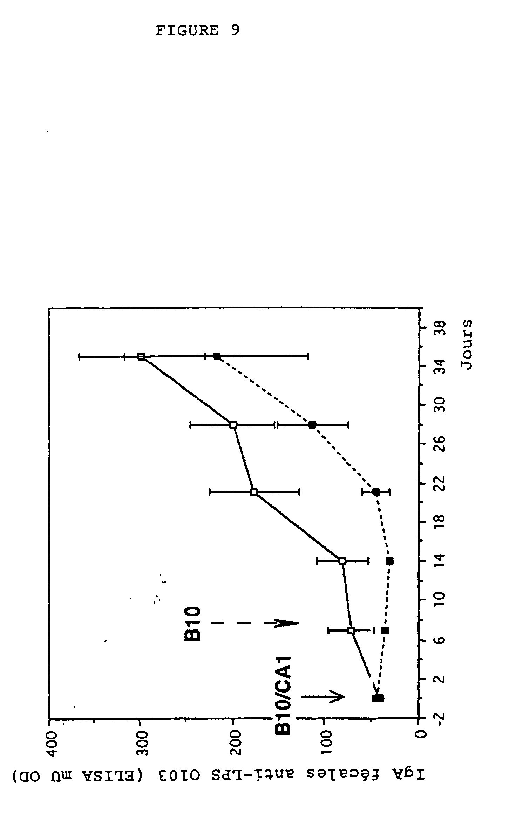

Parallèlement, les IgA locales anti-LPS 0103 ont été dosées par ELISA dans les fèces. La Figure 9 montre l'évolution des IgA fécales après inoculation orale de la souche ECP-négative B10/CA1, (au jour 0), puis de la souche virulente B10 (au jour 7) (□), ou bien de la seule inoculation de la souche virulente B10 au jour 7 (■). On observe que la réponse anticorps augmente significativement environ 15 jours après l'inoculation orale de la souche B10/CA1.

### EXEMPLE 6 : OBTENTION DE MUTANTS DU LEE PAR MUTAGENESE DIRIGEE

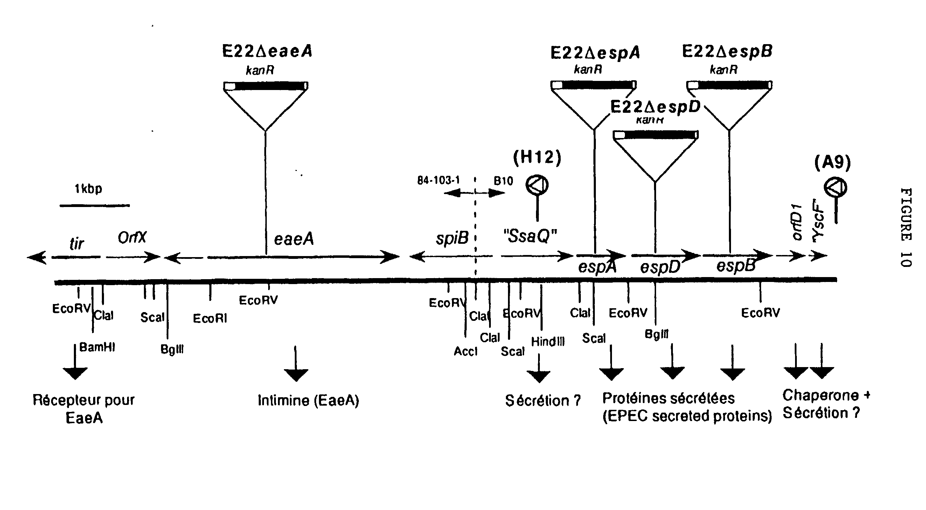

Les Inventeurs ont cloné et séquencé l'extrémité droite du LEE de la souche B10. L'organisation de la région séquencée est schématisée par la Figure 10, et la séquence obtenue est représentée dans la liste de séquences en annexe sous le numéro SEQ ID NO: 1. Les Inventeurs ont en outre étudié l'organisation générale du LEE chez les souches 0103 pathogènes, et ont confirmé qu'elle était identique chez toutes ces souches.

Les Inventeurs ont construit des mutants du LEE des souches O103 à partir de la souche dénommée E22. Cette souche pathogène est analogue à la souche B10, mais est sensible à la streptomycine (ainsi d'ailleurs qu'à à tous les antibiotiques à spectre Gram négatif testés).

Ces mutants ont été construits par la méthode de l'échange allélique.

Pour chacun des loci choisis (*eaeA, espA, espB,* et *espD*)*,* l'allèle de la souche sauvage E22 a été remplacé par un allèle muté par insertion d'une cassette [GALAN et al., J. Bacteriol., 174, 4338-4349, (1992)] contenant un gène de résistance à la kanamycine (gène *aphT:* aminoglycoside 3'phosphotransférase), sous contrôle d'un promoteur fort, et sans terminateur de transcription. Cette cassette, flanquée de séquences du gène à muter et portée par un plasmide suicide [KANIGA et al., Gene, 109, 137-141 (1991)], est introduite dans la souche sauvage, puis les bactéries recombinantes ayant remplacé l'allèle sauvage par l'allèle muté, sont sélectionnées.

Les gènes *eaeA* et *espD* ont été inactivés respectivement par insertion de la cassette dans les sites de restriction "naturels" EcoRV et *BglII.* Pour *espB* et *espA,* un site *BgIII* (GAGATC) a été *créé* de novo par mutagenèse dirigée (PCR à l'aide d'oligonucléotides dégénérés et sélection des plasmides par restriction):

Les protéines produites par les mutants obtenus ont été analysées par immunoempreintes pour EaeA et immunoprécipitation pour EspA, EspB et EspD. Ces analyses ont permis de vérifier que ces mutants ne différaient de la souche sauvage que par l'absence de production de la protéine dont le gène était inactivé par la mutation.
Les mutants obtenus sont les suivants :
- E22Δ*espA*, qui ne synthétise plus la protéine EspA.
- E22Δ*espB*, qui ne synthétise plus la protéine EspB.
- E22Δ*spD*, qui ne synthétise plus la protéine EspD.
- E22Δ*eaeA*, qui ne synthétise plus la protéine EaeA et
donc ne l'inclut plus dans sa membrane externe.

Pour chaque mutant, la synthèse des autres protéines demeure effectivement normale. Par exemple, le mutant *E22ΔespA* ne produit plus EspA, mais produit et secrète normalement EspB et EspD, de même qu'il synthétise et inclut normalement EaeA dans sa membrane externe. Il apparaît donc que ces mutants présentent une mutation stricte et non-polaire, concernant uniquement le gène cible de la mutation.

La Figure 10 représente l'extrémité droite du LEE des souches 0103, et indique la position des différents gènes, ainsi que l'emplacement d'insertion de la cassette pour les 4 mutants obtenus.

Ces mutants ont fait l'objet de tests *in vitro* visant à vérifier leur capacité à induire l'ECP sur cellules HeLa, décrit à l'exemple 1 ci-dessus.

Les mutants des gènes *esp* ont également été testés pour leur pouvoir pathogène chez le lapin sevré, par administration orale de 2 x 10⁷ CFU à des lots de lapereaux de 35 jours, selon le même protocole que celui décrit à l'Exemple 4 ci-dessus.

Les résultats obtenus sont résumés dans le Tableau I ci-dessous.

**TABLEAU I**

| Souche | ECP/HeLa | Pouvoir pathogène in vivo^{a} | | |
|---|---|---|---|---|
| | | Perte de poids | Diarrhée | Mortalité |
| E22* | +++ | 28/34 | 26/34 | 25/34 |
| E22Δ*espA* | - | 0/16 | 0/16 | 0/16 |
| E22Δ*espB* | - | 0/16 | 0/16 | 0/16 |
| E22Δ*espD* | - | 3/18 | 2/18 | 2/18 |
| E22Δ*eaeA* | +++ | NT | NT | NT |

| | | | | |
|---|---|---|---|---|
| a: nombre d'animaux ayant présenté le signe clinique/nombre d'animaux inoculés. | | | | |
| * :souche sauvage. NT: non testé. | | | | |

Ces résultats montrent que les mutants de synthèse des protéines Esp n'expriment plus l'effet cytopathique sur les cellules HeLa, contrairement au mutant de *eaeA.* En corollaire, deux des mutants des protéines Esp (*ΔespA* et *ΔespB*) ont complètement perdu leur pouvoir pathogène chez le lapin, et le pouvoir pathogène du mutant *ΔespD* est beaucoup plus faible que celui de la souche sauvage.

## Revendications

1. Procédé d'obtention d'une souche mutante non-pathogène d'*E. coli* à partir d'une souche d'*E. coli* pathogène capable d'induire sur des cellules épithéliales HeLa un effet cytopathique se manifestant par la formation de câbles d'actine polymérisée traversant de part en part lesdites cellules et par une augmentation de la quantité de vinculine, **caractérisé en ce que** l'on procède à la mutagenèse de ladite souche d'*E. coli* pathogène, et **en ce que** l'on sélectionne les mutants ayant perdu la capacité d'induire ledit effet cytopathique.

2. Souche mutante non-pathogène d'*E*. *coli* appartenant au sérogroupe O103, **caractérisée en ce qu'**elle est susceptible d'être obtenue par un procédé selon la revendication 1, à partir d'une souche *E. coli* du sérogroupe O103 pathogène du lapin, et **en ce qu'**elle possède un locus LEE dont au moins un des gènes est inactivé par la mutation provoquant la perte du pouvoir pathogène et de la capacité d'induire l'effet cytopathique.

3. Souche mutante selon la revendication 2, **caractérisée en ce qu'**elle est susceptible d'être obtenue à partir de la souche d'*E. coli* dénommée B10, déposée à la CNCM le 15 Janvier 1997 sous le n° I-1807.

4. Souche mutante non-pathogène d'*E. coli* selon une quelconque des revendications 2 ou 3, **caractérisée en ce qu'**elle porte au moins une mutation résultant en l'inactivation d'au moins l'un des gènes sep du locus LEE.

5. Souche mutante non-pathogène d'*E. coli* selon une quelconque des revendications 2 à 4, **caractérisée en ce qu'**elle possède une adhésine AF/R2 fonctionnelle.

6. Souche mutante non-pathogène d'*E. coli* dénommée B10/CA1, déposée auprès de la CNCM le 15 Janvier 1997 sous le n° I-1808.

7. Vaccin comprenant au moins une souche mutante non-pathogène d'*E. coli* selon une quelconque des revendications 2 à 6.

8. Vaccin selon la revendication 7, administrable par voie orale.

9. Utilisation d'une souche mutante d'*E. coli* selon une quelconque des revendications 2 à 6, pour l'obtention d'un vaccin.

10. Utilisation selon la revendication 9,
**caractérisée en ce que** ledit vaccin est destiné à être administré à des lapins.

## Patentansprüche

1. Verfahren zum Erhalt eines nicht-pathogenen Mutantenstamms von *E. coli* ausgehend von einem pathogenen *E. coli*-Stamm, der fähig ist, eine cytopathische Wirkung auf die HeLa-Epithelzellen zu induzieren, welche sich durch die Bildung von Strängen aus polymerisiertem Aktin, die die genannten Zellen durchdringen, und durch eine Erhöhung der Menge an Vinculin zeigt, **dadurch gekennzeichnet dass** man eine Mutagenese des pathogenen E. coli-Stamms vornimmt und dass man die Mutanten selektiert, die die Fähigkeit, die cytopathische Wirkung zu induzieren, verloren haben.

2. Nicht-pathogener Mutantenstamm von *E. coli*, der zur Serogruppe O103 gehört, **dadurch gekennzeichnet , dass** er durch ein Verfahren nach Anspruch 1, ausgehend von einem pathogenen *E. coli*-Stamm der Serogruppe O103 des Kaninchens erhalten werden kann und dass er einen Lokus LEE besitzt, von dem wenigstens eines der Gene durch die Mutation inaktiv ist, welche den Verlust pathogen zu sein und den Verlust des Vermögens, die cytopathische Wirkung zu induzieren, hervorruft.

3. Mutantenstamm nach Anspruch 2, **dadurch gekenn- zeichnet**, dass er, ausgehend von dem E. coli-Stamm mit der Bezeichnung B10, der bei der CNCM am 15. Januar 1997 unter der Nummer I-1807 hinterlegt wurde, erhalten werden kann.

4. Nicht-pathogener Mutantenstamm von E. coli nach einem der Ansprüche 2 oder 3, **dadurch gekennzeichnet , dass** er wenigstens eine Mutation trägt, die in der Inaktivierung wenigstens eines der Gene sep des Lokus LEE resultiert.

5. Nicht-pathogener Mutantenstamm von E. coli nach einem der Ansprüche 2-4, **dadurch gekennzeichnet , dass** er ein funktionelles Adhäsin AF/R2 besitzt.

6. Nicht-pathogener Mutantenstamm von E. coli mit der Bezeichnung B10/CA1, der bei der CNCM am 15. Januar 1997 unter der Nummer I-1808 hinterlegt wurde.

7. Vakzin, das wenigstens einen nicht-pathogenen Mutantenstamm von E. coli nach einem der Ansprüche 2-6 umfasst.

8. Vakzin nach Anspruch 7, das auf oralem Weg verabreichbar ist.

9. Verwendung eines Mutantenstamms von E. coli nach einem der Ansprüche 2-6 zum Erhalt eines Vakzins.

10. Verwendung nach Anspruch 9, **dadurch gekennzeich- net**, dass das Vakzin dazu bestimmt ist, Kaninchen verabreicht zu werden.

## Claims

1. Method of obtaining a non-pathogenic mutant strain of *E. coli* from a pathogenic strain of *E. coli* that is capable of inducing a cytopathic effect on HeLa epithelial cells, said effect appearing as the formation of cables of polymerized actin passing right through said cells, and as an increase in the amount of vinculin, **characterized in that** said pathogenic strain of *E. coli* is subjected to mutagenesis and **in that** the mutants that have lost the capacity to induce said cytopathic effect are selected.

2. Non-pathogenic mutant strain of *E. coli* belonging to serogroup O103, **characterized in that** it can be obtained by a method according to Claim 1 from a pathogenic rabbit strain of *E*. *coli* serogroup 0103, and **in that** it possesses an LEE of which at least one of the genes is inactivated by the mutation causing the loss of pathogenicity and of the capacity to induce the cytopathic effect.

3. Mutant strain according to Claim 2, **characterized in that** it can be obtained from the strain of *E. coli* called B10 deposited in the CNCM on 15 January 1997 under no. I-1807.

4. Non-pathogenic mutant strain of *E. coli* according to either Claim 2 or Claim 3, **characterized in that** it carries at least one mutation resulting in the inactivation of at least one of the *sep* genes of the LEE.

5. Non-pathogenic mutant strain of *E*. *coli* according to any one of Claims 2 to 4, **characterized in that** it possesses a functional adhesin AF/R2.

6. Non-pathogenic mutant strain of *E. coli* called B10/CA1 deposited in the CNCM on 15 January 1997 under no. I-1808.

7. Vaccine comprising at least one non-pathogenic mutant strain of *E. coli* according to any one of Claims 2 to 6.

8. Vaccine according to Claim 7 that can be administered orally.

9. Use of a mutant strain of *E. coli* according to any one of Claims 2 to 6 for obtaining a vaccine.

10. Use according to Claim 9, **characterized in that** said vaccine is intended to be administered to rabbits.
